(19) Europäisches Patentamt
European Patent Office
Office européen des brevets

(11) EP 2 998 294 A1

# (12) EUROPEAN PATENT APPLICATION

(43) Date of publication:
**23.03.2016 Bulletin 2016/12**

(21) Application number: **14306429.3**

(22) Date of filing: **16.09.2014**

(51) Int Cl.:
*C07D 213/71* (2006.01) *C07D 215/36* (2006.01)
*C07D 215/38* (2006.01) *C07D 231/12* (2006.01)
*C07D 231/18* (2006.01) *C07D 333/34* (2006.01)
*C07D 333/62* (2006.01) *C07C 209/30* (2006.01)
*C07C 311/21* (2006.01) *C07C 311/29* (2006.01)
*C07C 311/46* (2006.01) *C07D 265/36* (2006.01)
*C07D 207/27* (2006.01) *C07D 277/36* (2006.01)
*C07D 209/08* (2006.01) *C07D 277/46* (2006.01)
*C07D 277/62* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANOFI**
**75008 Paris (FR)**

(72) Inventor: **The designation of the inventor has not yet been filed**

(74) Representative: **Essler, Frank**
**Sanofi-Aventis Deutschland GmbH**
**Global Intellectual Property Department**
**Industriepark Höchst**
**Gebäude K 703**
**65926 Frankfurt am Main (DE)**

## (54) Naphthyl sulfonamide phenyl derivatives as KEAP-1 modulators for the treatment of diabetes, obesity, dyslipidemia and related disorders

(57) The present invention relates to naphthyl sulfonamide phenyl derivatives of the formula I

**(I)**

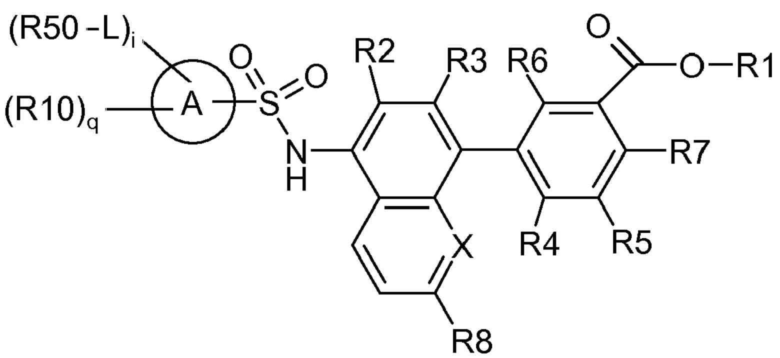

in which R1, R2, R3, R4, R5, R6, R7, R8, R10, A, X, L, q, i and R50 are defined as indicated below.

The naphthyl sulfonamide phenyl derivatives I are KEAP-1 modulators and useful for the prevention and/or treatment of diabetes, obesity, dyslipidemia and related disorders. The invention furthermore relates to the use of naphthyl sulfonamide phenyl derivatives of the formula I as active ingredients in pharmaceuticals, and pharmaceutical compositions comprising them.



Printed by Jouve, 75001 PARIS (FR)

## Description

The present invention relates to naphthyl sulfonamide phenyl derivatives of the formula I

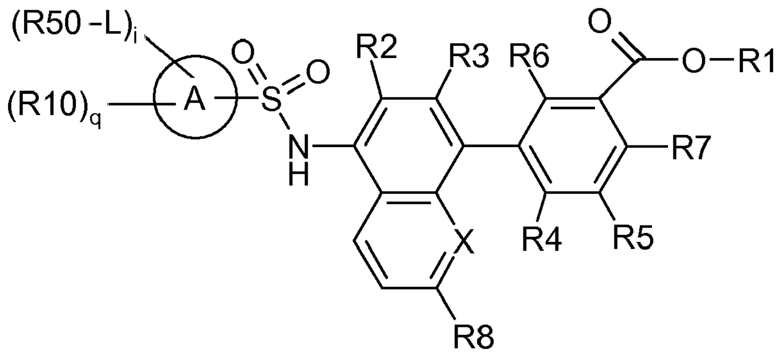

(I)

in which R1, R2, R3, R4, R5, R6, R7, R8, R10, A, X, L, q, i and R50 are defined as indicated below.

The naphthyl sulfonamide phenyl derivatives I are KEAP-1 modulators and useful for the prevention and/or treatment of diabetes, obesity, dyslipidemia and related disorders, neuroinflammatory and neurodegenerative diseases, such as multiple sclerosis, Alzheimer's disease, Parkinson's disease and Huntington's disease.The invention furthermore relates to the use of naphthyl sulfonamide phenyl derivatives of the formula I as active ingredients in pharmaceuticals, and pharmaceutical compositions comprising them.

Introduction

The transcription factor nuclear factor erythroid 2 p45 (NF-E2)-related factor (Nrf2) is a cellular sensor of oxidative and electrophilic stress. Nrf2 is a member of the Cap 'n' collar family of basic leucine zipper transcription factors and is critical for the expression of several cytoprotective genes. Nrf2 is regulated by the Kelch-like ECH-associated protein 1 (Keap1). Keap1 is an adaptor protein of the cullin 3-dependent ubiquitin E3 ligase complex. Under unstressed conditions Keap1 mediates the ubiquitinylation and subsequent degradation of Nrf2 by the 26S proteasome. Keap1 binds to Nrf2 in a stoichiometry of 2:1. The Keap1 dimer binds with its Kelch domains to both the DLG and the ETGE sequence motives of Nrf2. Keap1 contains in the so- called BTB- and IVR-domains highly reactive cysteine residues. In particular the cysteine residues at positions 151, 272 and 288 are modified in response to oxidative stress and by electrophilic reagents. This inhibits Keap1 function and prevents the degradation of Nrf2. Subsequently Nrf2 translocates to the nucleus, where it binds as a heterodimer with small Maf proteins to the so-called anti-oxidant response element (ARE), the promoter region of its target genes. Protein kinases such as extracellular-signal-regulated kinase (ERK), p38 mitogen-actived protein kinase (MAPK), phosphoinositide 3-kinase (PI3K), protein kinase C (PKC) and glycogen synthase kinase-3 (GSK3) can also modulate the activity of Nrf2. Pharmacological activators of Nrf2 stimulates the expression of several cytoprotective genes involved in Phase II metabolism and oxidative-defence pathways. Furthermore, Nrf2 has been linked to an upregulation of mitochondrial biogenesis and fat oxidation. The inhibition of Nrf2 and subsequent stimulation of Nrf2 also prevents the activation of macrophages by interferon. With that, the keap1/Nrf2 signalling also controls inflammatory processes. Target genes of Nrf2 include NAD(P)H:quinone-oxidoreductase-1 (NQO1) and heme oxygenase 1 (HMOX1), which are upregulated by the activation of Nrf2. Activators such as oleanane triterpenoids, dimethyl fumarate and sufloraphane possess reactive functional groups that activate Nrf2 by the modification of the reactive cysteine-residues within the BTB- and IVR-domains of Keap1. Such reactive molecules also react with reactive cysteine residues of other proteins. Mutational analyses have shown that Nrf2 can also be activated by the selective inhibition of the protein-protein interaction between Nrf2 and the kelch domain of Keap1. The activation of Nrf2 triggers a pleiotropic biological response and has drawn high medical interest as a potential approach for the prevention and treatment of diseases associated with increased oxidative and inflammatory stress. In diabetics the activation of Nrf2 is impaired, resulting in a reduced oxidative-stress defence. Diabetes is also associated with low-grade inflammation. Inflammatory processes and excessive oxidative stress induce insulin resistance, endothelial dysfunction and late-stage diabetic complications, such as diabetic nephropathy. With that, pharmacological Nrf2 activation has the potential to improve glucose control, lipid handling and also micro- and macrocardiovascular disease in diabetics. The activation of Nrf2 reduces cellular stress and damage in response to xenobiotics, indicating that this approach could be useful for the prevention of cancer. The excessive production of reactive oxidative species contributes to neurodegenerative

diseases such as multiple sclerosis, Huntington's and Parkinson's disease, indicating that the activation of Nrf2 is a potential therapeutic strategy for these diseases. Targeting this transcription factor is also useful for chronic inflammatory diseases such as chronic obstructive pulmonary disease, arthritis and sepsis.

Several modulators of KEAP-1 are known, see for example D. Marcotte et al, Bioorganic and Medical Chemistry 21, 2013, 4011-4019, Zhuang et al, J. Med. Chem. 57, 2014, 1121-1126 and WO2013067036. Naphthyl sulfonamides are also described in CN101836971A for the treatment of cancer and psoriasis.

It was an aim of the invention to provide novel compounds as active ingredients in pharmaceuticals.

It was an aim of the invention to provide novel compounds which will lower blood glucose in mammals and which are suitable for prevention and/or treatment of diabetes, obesity, dyslipidemia and related disorders.

A further aim was to provide novel KEAP-1 modulators, especially antagonists, which can be used therapeutically for the prevention and/or treatment of diabetes, obesity, dyslipidemia and related disorders.

Accordingly a subject of the invention is a compound of the formula I

**(I)**

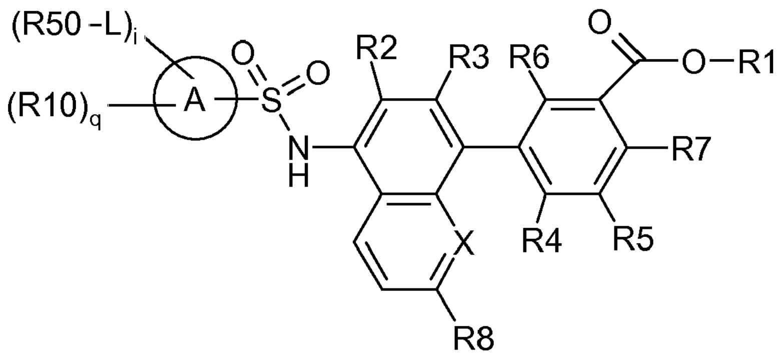

in which

| | |
|---|---|
| R1 | is H or $(C_1-C_6)$-alkyl; |
| A | is phenyl, a 5-7 membered heteroaryl, a 9-11 membered heteroaryl or a 9 to 11 membered heterobicycle; |
| X | is CH or N; |
| R2 | is H, $(C_1-C_{16})$-alkyl or O-$(C_1-C_{16})$-alkyl; |
| R3 | is H, $(C_1-C_{16})$-alkyl or O-$(C_1-C_{16})$-alkyl; |
| R4, R5, R6, R7 | are independently of one another H, F, Cl, Br, I, OH, $CF_3$, $NO_2$, CN, $OCF_3$, O$(C_1-C_6)$-alkyl, O-$(C_1-C_4)$-alkoxy-$(C_1-C_4)$-alkyl, S-$(C_1-C_6)$-alkyl, $(C_1-C_6)$-alkyl, N(R11)(R12), $SO_2$-$CH_3$, N(R13)CO(R14), N(R15)$SO_2$(R16) or CO(R17); |
| R8 | is H or $(C_1-C_6)$-alkyl; |
| R10 | is independently of each other F, Cl, Br, I, OH, $CF_3$, $NO_2$, CN, $OCF_3$, O-$(C_1-C_6)$-alkyl, O-$(C_1-C_4)$-alkoxy-$(C_1-C_4)$-alkyl, S-$(C_1-C_6)$-alkyl, $(C_1-C_8)$-alkyl, $(C_2-C_6)$-alkenyl, $(C_2-C_6)$-alkynyl, N(R18)(R19), $SO_2$-$CH_3$, COOH, COO-$(C_1-C_6)$-alkyl, CON(R20)(R21), N(R22)CO(R23), N(R24)$SO_2$(R25), CO(R26), $(C(R27)(R28))_z$-O(R29) or oxo; |
| z | is 0, 1, 2, 3, 4, 5 or 6; |
| R11, R12, R13, R14, R15, R16, R17, R18, R19, R20, R21, R22, R23, R24, R25, R26,R27, R28, R29 | are independently of one another H or $(C_1-C_6)$-alkyl; |
| q | is 0, 1, 2, 3 or 4; |
| L | is a bond, NH, O, CO, NHCO or CONH; |

(continued)

| | |
|---|---|
| R50 | is phenyl, a 5-6 membered heteroaryl, a 5-6 membered heterocyclyl or $(C_3-C_8)$-cycloalkyl, wherein R50 is optionally substituted one or more times independently of one another by F, Cl, Br, I, OH, $CF_3$, $NO_2$, CN, $OCF_3$, O-$(C_1-C_6)$-alkyl, O-$(C_1-C_4)$-alkoxy-$(C_1-C_4)$-alkyl, S-$(C_1-C_6)$-alkyl, $(C_1-C_6)$-alkyl, $(C_2-C_6)$-alkynyl, N(R31)(R32), $SO_2$-$CH_3$, COOH, COO-$(C_1-C_6)$-alkyl, CON(R33)(R34), N(R35)CO(R36), N(R37)$SO_2$(R38), CO(R39), $(C(R40)(R41))_y$-O(R42) or oxo; |
| R31, R32, R33, R34, R35, R36, R37, R38, R39, R40, R41, R42 | are independently of one another H or $(C_1-C_6)$-alkyl; |
| y | 0, 1, 2 ,3, 4, 5 or 6; |
| i | is 0 or 1; |

in any of its stereoisomeric forms, or a mixture of stereoisomeric forms in any ratio, or a physiologically acceptable salt thereof, or a physiologically acceptable solvate of any of them.

In another group of embodiments of a compound of the formula I

A is phenyl.

In another group of embodiments of a compound of the formula I

A is a 5-7 membered heteroaryl.

In another group of embodiments of a compound of the formula I

A is a 5-7 membered heteroaryl selected from the group of thiazol, pyrazol, thienyl, pyridyl and furyl.

In another group of embodiments of a compound of the formula I

A is a 9 to 11 membered heteroaryl selected from the group of benzothiophen, 1,3-benzothiazol, imidazo[2,1b]thiazol and indol.

In another group of embodiments of a compound of the formula I

A is a 9 to 11 membered heterobicycle.

In another group of embodiments of a compound of the formula I

A is a 9 to 11 membered heterobicycle selected from the group of indolin, dihydro-2H-quinolin, dihydro-1,4-benzoxazin,1,3-benzothiazol and 2,3-dihydro-1,4-benzodioxin.

In another group of embodiments of a compound of the formula I

A is selected from the group of phenyl, thiazol, pyrazol, thienyl, pyridyl, furyl, benzothiophen, 1,3-benzothiazol, imidazo[2,1b]thiazol, indol, indolin, di hydro-2H-quinolin, dihydro-1,4-benzoxazin, 2,3-dihydro-1,4-benzodioxin and tetraline.

In another group of embodiments of a compound of the formula I

| | |
|---|---|
| R10 | is independently of each other F, Cl, Br, I, OH, $CF_3$, $NO_2$, CN, $OCF_3$, O-$(C_1-C_6)$-alkyl, O-$(C_1-C_4)$-alkoxy-$(C_1-C_4)$-alkyl, S-$(C_1-C_8)$-alkyl, $(C_1-C_6)$-alkyl, $(C_2-C_6)$-alkenyl, $(C_2-C_6)$-alkynyl, N(R18)(R19), $SO_2$-$CH_3$, COOH, COO-$(C_1-C_6)$-alkyl, CON(R20)(R21), N(R22)CO(R23), N(R24)$SO_2$(R25), CO(R26), $(C(R27)(R28))_z$-O(R29) or oxo; |
| z | is 0, 1, 2, 3, 4, 5 or 6; |

(continued)

| | |
|---|---|
| R18, R19, R20, R21, R22, R23, R24, R25, R26, R27, R28, R29 | are independently of one another H or ($C_1$-$C_6$)-alkyl. |

In another group of embodiments of a compound of the formula I

| | |
|---|---|
| R10 | is independently of each other F, Cl, OH, $CF_3$, CN, $OCF_3$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_8$)-alkyl, $SO_2$-$CH_3$, COOH, COO-($C_1$-$C_6$)-alkyl, CON(R20)(R21), N(R22)CO(R23), CO(R26), $(C(R27)(R28))_z$-O(R29) or oxo; |
| z | is 0, 1, 2, 3, 4, 5 or 6; |
| R20, R21, R22, R23, R26, R27, R28, R29 | are independently of one another H or ($C_1$-$C_6$)-alkyl. |

In another group of embodiments of a compound of the formula I

| | |
|---|---|
| R10 | is independently of each other F, Cl, Br, I, OH, $CF_3$, CN, $OCF_3$, O-($C_1$-$C_6$)-alkyl, ($C_1$-$C_8$)-alkyl, COOH, COO-($C_1$-$C_6$)-alkyl, N(R22)CO(R23), CO(R26), $(C(R27)(R28))_z$-O(R29) or oxo; |
| z | is 0, 1, 2, 3 or 4; |
| R22, R23, R26, R27, R28, R29 | are independently of one another H or ($C_1$-$C_6$)-alkyl. |

In another group of embodiments of a compound of the formula I

R10 is independently of each other F, Cl, methyl, propyl, butyl, isobutyl, octyl, COOH, $NHCOCH_3$, $OCH_3$, CN, $COCH_3$, $CF_3$ or oxo.

In another group of embodiments of a compound of the formula I

X is CH.

In another group of embodiments of a compound of the formula I

X is N.

In another group of embodiments of a compound of the formula I

R2 is H;
R3 is H.

In another group of embodiments of a compound of the formula I

R2 is ($C_1$-$C_{16}$)-alkyl or O-($C_1$-$C_{16}$)-alkyl;
R3 is H.

In another group of embodiments of a compound of the formula I

| | |
|---|---|
| R4, R5, R6 and R7 | independently of one another H, F, Cl, OH, $CF_3$, CN, $OCF_3$, O-($C_1$-$C_6$)-alkyl, ($C_1$-$C_6$)-alkyl, N(R11)(R12), $SO_2$-$CH_3$, N(R13)CO(R14), N(R15)$SO_2$(R16) or CO(R17); |
| R11, R12, R13, R14, R15, R16, R17 | are independently of one another H or ($C_1$-$C_6$)-alkyl. |

In another group of embodiments of a compound of the formula I

R4, R5, R6 and R7 independently of one another H, F, Cl, OH, $CF_3$, CN, $OCF_3$, O-($C_1$-$C_6$)-alkyl or ($C_1$-$C_6$)-alkyl.

In another group of embodiments of a compound of the formula I
R4 is F;
R5, R6 and R7 are independently of one another H, F, Cl, OH or ($C_1$-$C_6$)-alkyl.
In another group of embodiments of a compound of the formula I
R4 is F;
R5, R6 and R7 are H.
In another group of embodiments of a compound of the formula I
R4, R5, R6 and R7 are H.
In another group of embodiments of a compound of the formula I

L is a bond, NH, O, CO, NHCO or CONH.

In another group of embodiments of a compound of the formula I

L is a bond, NH, NHCO or CONH.

In another group of embodiments of a compound of the formula I

L is a bond.

In another group of embodiments of a compound of the formula I

R50 is phenyl, a 5-6 membered heteroaryl, a 5-6 membered heterocyclyl or ($C_3$-$C_8$)-cycloalkyl, wherein R50 is optionally substituted one or more times independently of one another by F, Cl, OH, $CF_3$, $NO_2$, CN, $OCF_3$, O-($C_1$-$C_6$)-alkyl, ($C_1$-$C_6$)-alkyl, COO-($C_1$-$C_6$)-alkyl, CO(R39) or oxo;

R39 is H or ($C_1$-$C_6$)-alkyl;

In another group of embodiments of a compound of the formula I

R50 is phenyl, a 5-6 membered heteroaryl, a 5-6 membered heterocyclyl or ($C_3$-$C_8$)-cycloalkyl, wherein R50 is optionally substituted one or more times independently of one another by F, Cl, $CF_3$, O-($C_1$-$C_6$)-alkyl, ($C_1$-$C_6$)-alkyl, COO-($C_1$-$C_6$)-alkyl, CO(R39) or oxo;

R39 is H or ($C_1$-$C_6$)-alkyl.

In another group of embodiments of a compound of the formula I

R50 is phenyl, a 5-6 membered heteroaryl, a 5-6 membered heterocyclyl or ($C_3$-$C_8$)-cycloalkyl, wherein R50 is optionally substituted one or more times independently of one another by F, Cl, $CF_3$, ($C_1$-$C_6$)-alkyl or oxo.

In another group of embodiments of a compound of the formula I

R50 is phenyl, wherein R50 is optionally substituted one or more times independently of one another by F, Cl, $CF_3$ or ($C_1$-$C_6$)-alkyl.

In another group of embodiments of a compound of the formula I

R50 is a 5-6 membered heteroaryl, wherein R50 is optionally substituted one or more times independently of one another by F, Cl, $CF_3$ or ($C_1$-$C_6$)-alkyl.

In another group of embodiments of a compound of the formula I

R50 is a 5-6 membered heterocyclyl, wherein R50 is optionally substituted one or more times independently of one

another by F, Cl, $CF_3$, $NO_2$, $(C_1-C_6)$-alkyl, COO-$(C_1-C_6)$-alkyl or oxo.

In another group of embodiments of a compound of the formula I

R50 is $(C_3-C_8)$-cycloalkyl, wherein R50 is optionally substituted one or more times independently of one another by F, Cl, $CF_3$, $NO_2$, $(C_1-C_6)$-alkyl, COO-$(C_1-C_6)$-alkyl or oxo.

In another group of embodiments of a compound of the formula I

R50 is selected from the group of phenyl, pyrazol, pyrrolidin, morpholin and cyclopentyl, wherein R50 is optionally substituted once by $CF_3$ or oxo.

In another group of embodiments of a compound of the formula I
i is 0; and
A is a 9 to 11 membered heterobicycle.
In another group of embodiments of a compound of the formula I
i is 0; and
A is phenyl or a 9 to 11 membered heteroaryl.
In another group of embodiments of a compound of the formula I
i is 1; and
A is phenyl or a 5 to 7 membered heteroaryl.
In another group of embodiments of a compound of the formula I
i is 0; and
A is phenyl or a 5 to 7 membered heteroaryl.
Another embodiment is a compounds of formula I in which

R1 is H or $(C_1-C_6)$-alkyl;

A is selected from the group of phenyl, thiazol, pyrazol, thienyl, pyridyl, furyl, benzothiophen, 1,3-benzothiazol, imidazo[2,1b]thiazol, indol, indolin, di hydro-2H-quinolin, dihydro-1,4-benzoxazin, 2,3-dihydro-1,4-benzodioxin and tetraline;

| | |
|---|---|
| X | is CH or N; |
| R2 | is H or $(C_1-C_8)$-alkyl; |
| R3 | is H or $(C_1-C_8)$-alkyl; |
| R4, R5, R6 and R7 | independently of one another H, F, Cl, OH, $CF_3$, CN, $OCF_3$, O-$(C_1-C_6)$-alkyl or $(C_1-C_6)$-alkyl; |
| R8 | is H or $(C_1-C_6)$-alkyl; |
| R10 | is independently of each other F, Cl, Br, I, OH, $CF_3$, CN, $OCF_3$, O-$(C_1-C_6)$-alkyl, $(C_1-C_8)$-alkyl, COOH, COO-$(C_1-C_6)$-alkyl, N(R22)CO(R23), CO(R26), $(C(R27)(R28))_z$-O(R29) or oxo; |
| z | is 0, 1, 2, 3 or 4; |
| R22, R23, R26, R27, R28, R29 | are independently of one another H or $(C_1-C_6)$-alkyl. |
| q | is 0, 1, 2, 3 or 4; |
| L | is a bond, NHCO or CONH; |
| R50 | is selected from the group of phenyl, pyrazol, pyrrolidin, morpholin and cyclopentyl, wherein R50 is optionally substituted once by $CF_3$ or oxo; |

i is 0 or 1;

in any of its stereoisomeric forms, or a mixture of stereoisomeric forms in any ratio, or a physiologically acceptable salt thereof, or a physiologically acceptable solvate of any of them.

In another embodiment compounds of the formula I are encompassed selected from the following list:

4-fluoro-3-[4-[(2,3,5,6-tetramethylphenyl)sulfonylamino]-1-naphthyl]benzoic acid,
3-[4-[(2,3,5,6-tetramethylphenyl)sulfonylamino]-1-naphthyl]benzoic acid,
2-fluoro-3-[4-[(2,3,5,6-tetramethylphenyl)sulfonylamino]-1-naphthyl]benzoic acid, 3-[4-[(5-chloro-1,3-dimethyl-pyrazol-4-yl)sulfonylamino]-1-naphthyl]-4-fluoro-benzoic acid,
3-[4-[(5-chloro-1,3-dimethyl-pyrazol-4-yl)sulfonylamino]-1-naphthyl]benzoic acid,
4-fluoro-3-[4-[(1,3,5-trimethylpyrazol-4-yl)sulfonylamino]-1-naphthyl]benzoic acid,
4-fluoro-3-[2-methyl-5-[(2,3,5,6-tetramethylphenyl)sulfonylamino]-8-quinolyl]benzoic acid; 2,2,2-trifluoroacetic acid,
3-[4-[(2,5-dichloro-3,6-dimethyl-phenyl)sulfonylamino]-1-naphthyl]benzoic acid,
3-[4-[(1-butyl-3-methyl-pyrazol-4-yl)sulfonylamino]-1-naphthyl]benzoic acid,
3-[4-[[2,6-dichloro-4-(trifluoromethyl)phenyl]sulfonylamino]-1-naphthyl]-4-fluoro-benzoic acid,
3-[4-[(1,3,5-trimethylpyrazol-4-yl)sulfonylamino]-1-naphthyl]benzoic acid,
3-[4-[(2,3-dichlorophenyl)sulfonylamino]-1-naphthyl]-4-fluoro-benzoic acid,
3-[4-[(5-chloro-2-methyl-phenyl)sulfonylamino]-1-naphthyl]-4-fluoro-benzoic acid,
3-[4-[(2,4-dichloro-5-methyl-phenyl)sulfonylamino]-1-naphthyl]-4-fluoro-benzoic acid and
3-[5-[(1,2-dimethylindol-3-yl)sulfonylamino]-8-quinolyl]-4-fluoro-benzoic acid;

in any of its stereoisomeric forms, or a mixture of stereoisomeric forms in any ratio, or a physiologically acceptable salt thereof, or a physiologically acceptable solvate of any of them.

Embodiments of a compound of formula I are also those which are generated by combinations of embodiments listed above.

Structural elements such as groups, substituents, hetero ring members, numbers or other features, for example alkyl groups, groups like R1, R2, R3 etc., which can occur several times in the compounds of the formula I, can all independently of one another have at each occurrence any of the indicated meanings and can in each case be identical to or different from one another. For example, the alkyl groups in a dialkylamino group can be identical or different.

Herein, the terms "including" and "comprising" are used in their open, non-limiting sense. As used herein, the terms "($C_1$-$C_6$)" and so forth refer to moieties having 1 to 6 carbon atoms and so forth, respectively. Within composed terms like "hydroxy-($C_0$-$C_4$)-alkyl" the option "($C_0$)-alkyl refers to a bond (i.e. in this case a directly bound hydroxy group), or in case of an unsubstituted "($C_0$)-alkyl" it refers to a hydrogen.

The term "alkyl", as used herein, refers to saturated, monovalent hydrocarbon radicals. The term "alkenyl", as used herein, refers to monovalent hydrocarbon radicals, which contain at least one carbon-carbon double bond, wherein each double bond can have E- or Z-configuration. The term "alkynyl", as used herein, refers to monovalent hydrocarbon radicals, which contain at least one carbon-carbon triple bond. The alkyl, alkenyl and alkynyl groups can be linear, i.e. straight-chain, or branched. This also applies when they are part of other groups, for example alkyloxy groups (= alkoxy groups, O-alkyl groups), alkyloxycarbonyl groups or alkyl-substituted amino groups, or when they are substituted. Depending on the respective definition, the number of carbon atoms in an alkyl group can be 1, 2, 3, 4, 5 or 6, or 1, 2, 3, or 4. Examples of alkyl are methyl, ethyl, propyl including n-propyl and isopropyl, butyl including n-butyl, sec-butyl, isobutyl and tert-butyl, pentyl including n-pentyl, 1-methylbutyl, isopentyl, neopentyl and tert-pentyl, hexyl including n-hexyl, 3,3-dimethylbutyl and isohexyl. Double bonds and triple bonds in alkenyl groups and alkynyl groups respectively can be present in any positions. Examples of alkenyl and alkynyl are ethenyl, prop-1-enyl, prop-2-enyl (= allyl), but-2-enyl, 2-methylprop-2-enyl, 3-methylbut-2-enyl, hex-3-enyl, hex-4-enyl, prop-2-ynyl (= propargyl), but-2-ynyl, but-3-ynyl, hex-4-ynyl or hex-5-ynyl. Substituted alkyl groups, alkenyl groups and alkynyl groups can be substituted in any positions, provided that the respective compound is sufficiently stable and is suitable for the desired purpose such as use as a drug substance. The prerequisite that a specific group and a compound of the formula I are sufficiently stable and suitable for the desired purpose such as use as a drug substance, applies in general with respect to the definitions of all groups in the compounds of the formula I.

Independently of one another and independently of any other substituents, alkyl groups, divalent alkyl groups, alkenyl groups, alkynyl groups, cycloalkyl groups and heterocycloalkyl groups are optionally substituted by one or more fluorine substituents which can be located in any positions, i.e., the said groups can be unsubstituted by fluorine substituents or substituted by fluorine substituents, for example by 1, 2 or 3, by 1 or 2, or by 1 fluorine substituents. Examples of fluorine-substituted said groups are trifluoromethyl, difluoromethyl and fluoromethyl.

The term "alkanediyl" or "alkylene" ", as used herein, refers to saturated, divalent hydrocarbon radicals. The term "alkenediyl", as used herein, refers to divalent hydrocarbon radicals, which contain at least one carbon-carbon

double bond, wherein each double bond can have E- or Z-configuration. The term "alkyndiyl", as used herein, refers to divalent hydrocarbon radicals, which contain at least one carbon-carbon triple bond. As far as applicable, the preceding explanations regarding alkyl, alkenyl and alkynyl groups apply correspondingly to alkanediyl, alkendiyl and alkyndiyl groups, which thus can likewise be linear and branched. Examples of divalent alkyl groups are - $CH_2$- (= methylene), -$CH_2$-$CH_2$-, -$CH_2$-$CH_2$-$CH_2$-, -$CH_2$-$CH_2$-$CH_2$-$CH_2$-, -$CH(CH_3)$-, - $C(CH_3)_2$-, -$CH(CH_3)$-$CH_2$-, -$CH_2$-$CH(CH_3)$-, -$C(CH_3)_2$-$CH_2$- and -$CH_2$-$C(CH_3)_2$-.

The term "cycloalkyl", as used herein, unless otherwise indicated, refers to a monovalent radical of a saturated hydrocarbon ring system, which is monocyclic. In a monocyclic cycloalkyl group the number of ring carbon atoms can be for example 3, 4, 5, 6, 7 or 8. In one embodiment of the invention, the number of ring carbon atoms in a cycloalkyl group, independently of the number of ring carbon atoms in any other cycloalkyl group is 3, 4, 5 or 6, in another embodiment 3 or 4, in another embodiment 3, in another embodiment 5 or 6, in another embodiment 5, in another embodiment 6. Examples of cycloalkyl groups are cyclopropyl, cyclobutyl, cyclopentyl and cyclohexyl. The term "heterocycle", as used herein, unless otherwise indicated, refers to a cycloalkyl as defined above, in which 1, 2, 3 or 4 carbon atoms are replaced by nitrogen or oxygen atoms, provided that the heterocycloalkyl system is stable and suitable as a subgroup for the desired purpose of the compound of the formula I such as use as a drug substance. Depending on the definition of the respective heterocyclic group, in one embodiment of the invention the number of ring heteroatoms which can be present in a heterocyclic group, independently of the number of ring heteroatoms in any other heterocyclic group, is 1 or 2, in another embodiment 2, in another embodiment 1, wherein the ring heteroatoms can be identical or different. The heterocycloalkyl group can be attached by any ring carbon atom or saturated ring nitrogen atom, with the exception of spiro- or bridgehead atoms.

The term "aryl", as used herein, refers to a radical derived from an aromatic hydrocarbon by removal of one hydrogen, such as phenyl.

The term "heteroaryl" or "hetaryl" as used herein, refers to a radical derived from an aromatic mono- or bicyclic ring system, in which 1, 2, 3, 4 or 5 carbon atoms are replaced by heteroatoms.
5 to 7 membered heteroaryl means a monocyclic ring, 9 to 11 membered heteroaryl means a bicyclic ringsystem. The ring heteroatoms are generally chosen from N, O and S, wherein N includes ring nitrogen atoms which carry a hydrogen atom or a substituent as well as ring nitrogen atoms which do not carry a hydrogen atom or a substituent. Ring heteroatoms can be located in any position, provided that the heterocyclic system is stable and suitable as a subgroup for the desired purpose of the compound of the formula I such as use as a drug substance. Heteroaryl radicals are derived from 5-membered or 6-membered monocyclic rings or 9-membered or 10-membered bicyclic rings, in another embodiment 5-membered or 6-membered monocyclic rings or 9-membered or 10-membered bicyclic rings, in another embodiment 5-membered or 6-membered monocyclic rings.

Exemplary heteroaryl systems are derived from, but not limited to, the following ring systems: pyrrole, furan, thiophene, imidazole, pyrazole, oxazole (= [1,3]oxazole), isoxazole (= [1,2]oxazole), thiazole (= [1,3]thiazole), isothiazole (= [1,2]thiazole), [1,2,3]triazole, [1,2,4]triazole, [1,2,4]oxadiazole, [1,3,4]oxadiazole, [1,2,4]thiadiazole, [1,3,4]thiadiazole, tetrazole, pyridine, pyridazine, pyrimidine, pyrazine, [1,2,3]triazine, [1,2,4]triazine, [1,3,5]triazine, indole, isoindole, benzofuran, benzothiophene [1,3]benzoxazole, [1,3]benzothiazole, benzoimidazole,indazole, quinoline, isoquinoline, cinnoline, quinazoline, quinoxaline, phthalazine, different naphthyridines, e.g. [1,8]naphthyridine, different thienopyridines, e.g. thieno[2,3-b]pyridine and purine:

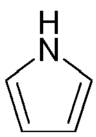 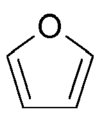 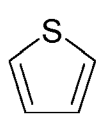 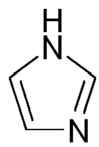 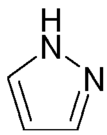 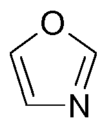 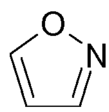 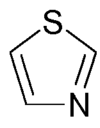 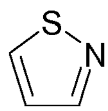

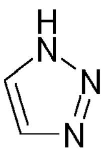 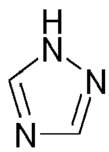 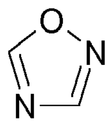 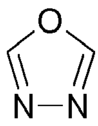 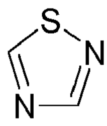 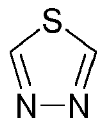 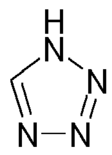

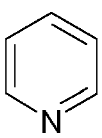 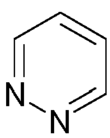 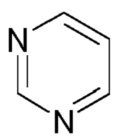 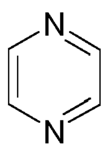 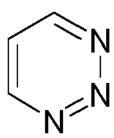 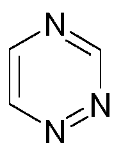 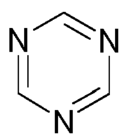

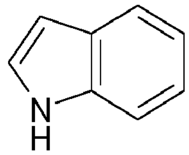 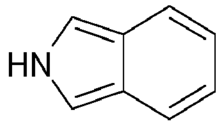 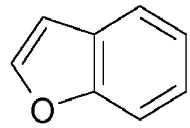 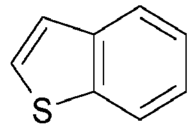

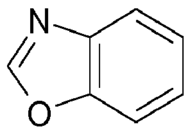 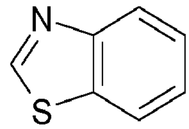 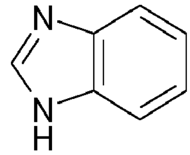 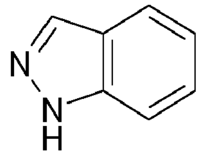

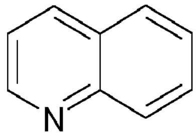 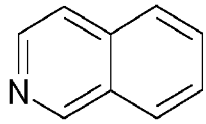 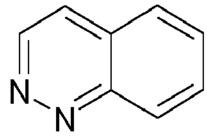 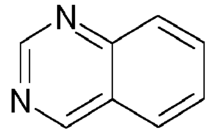

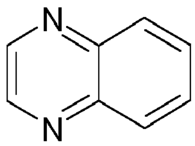 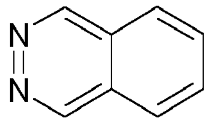 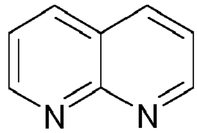 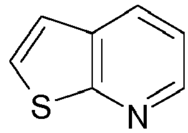 and 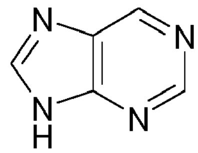

"9 to 11 membered heterobicyclyl" or "9 to 11 membered heterobicycle" means a heterocyclic system of two rings with 9 to 11 ring atoms, where one ring is phenyl, and wherein 2 ring atoms are shared by both rings and the other ring may contain one or more double bonds (to result in a non-aromatic ring which is partially saturated or un-saturated) and wherein at least one ring atom up to 5 ring atoms are replaced by a heteroatom selected from the group consisting of sulfur (including -S(O)-, $-S(O)_2-$), oxygen and nitrogen (including =N(O)-) and wherein the phenyl ring is linked to the $SO_2$ group of formula I. Examples for a 9 to 11 membered heterobicycle indoline, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine or pteridine.

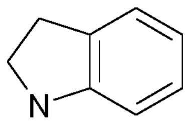 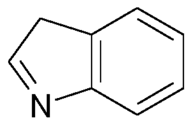 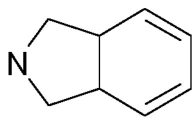 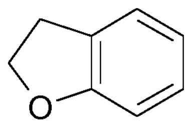 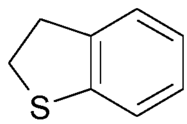

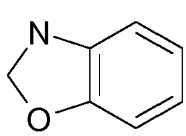 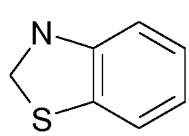 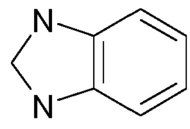 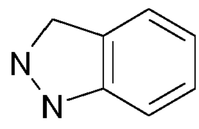

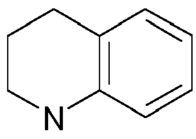 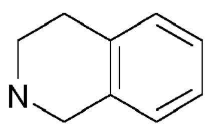 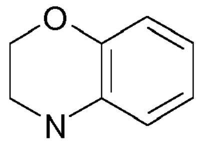 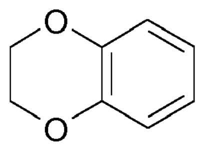

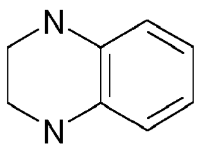 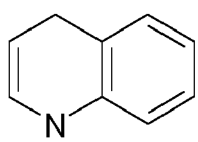 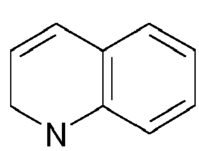

5 to 7 membered heterocyclyl" or "5 to 7 membered heterocycle" means a ring with 5, 6 or 7 ring atoms that may contain double bonds (non-aromatic ring which is fully, partially or un-saturated) wherein at least one ring atom up to 4 ring atoms are replaced by a heteroatom selected from the group consisting of sulfur (including -S(O)-, $-S(O)_2-$), oxygen and nitrogen (including =N(O)-) and wherein the ring is linked to the rest of the molecule via a carbon or nitrogen atom. Examples for a 5 to 7 membered heterocycles are pyrroline, pyrazoline, oxazoline, isoxazoline, thiazoline, isothiazoline, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridazine, piperazine, piperidine, morpholine, triazolidine, tetrazolidine, diazepane, azepine or homopiperazine.

Exemplary monocyclic heterocycloalkyl groups are derived from, but not limited to, the ring systems pyrrolidine, tetrahydrofuran, 1,3-dioxolane, piperidine, piperazine, morpholine, tetrahydropyran or 1,4-dioxane:

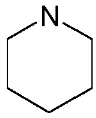 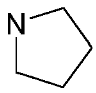 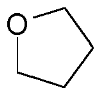 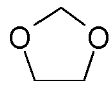

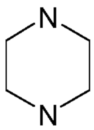 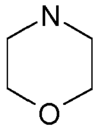 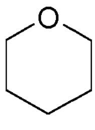 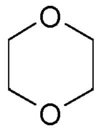

Groups like phenyl and residues of aromatic heterocycles which are optionally substituted by one or more substituents, can be unsubstituted or substituted, for example by 1, 2 or 3, or by 1 or 2, or by 1, identical or different substituents which can be located in any positions. Aromatic nitrogen heterocycles which in the parent ring system carry a hydrogen atom on a ring nitrogen atom in a 5-membered ring, such as a pyrrole or imidazole ring, for example, can be substituted on ring carbon atoms and/or on such ring nitrogen atoms. In one embodiment of the invention, substituents on such ring nitrogen atoms are chosen from $(C_1-C_4)$-alkyl groups, i.e. such ring nitrogen atoms in aromatic heterocycles carry a hydrogen atom or a $(C_1-C_4)$-alkyl substituent. When it is stated with respect to ring nitrogen atoms in aromatic heterocycles and any other heterocycles that they can carry a hydrogen atom or a substituent, such ring nitrogen atoms either carry a hydrogen atom or a substituent or they do not carry a hydrogen atom or substituent. Ring nitrogen atoms which carry a hydrogen atom or a substituent, occur in a nitrogen-containing aromatic 5-membered ring as is present in pyrrole or imidazole for example, and in a non-aromatic ring including a saturated ring. Ring nitrogen atoms which do not carry a hydrogen atom or a substituent unless they are present in positively charged form, including any further ring nitrogen atoms in addition to ring nitrogen atoms which carry a hydrogen atom or a substituent, occur in an aromatic ring as is present in thiazole, imidazole or pyridine, for example, and in a non-aromatic ring in which they are part of a double bond, and they occur as ring nitrogen atoms via which a ring is bonded. Suitable ring nitrogen atoms in aromatic heterocycles in the compounds of the formula I, such as the ring nitrogen atom in a pyridine ring, can in general also be present as N-oxide or as quaternary salt, for example as N-$(C_1-C_4)$-alkyl salt such as N-methyl salt, wherein in one embodiment of the invention the counter anion in such quaternary salt is a physiologically acceptable anion which is derived from an acid that forms a physiologically acceptable salt.

In monosubstituted phenyl groups, the substituent can be located in the 2-position, the 3-position or the 4-position. In disubstituted phenyl groups, the substituents can be located in 2,3-position, 2,4-position, 2,5-position, 2,6-position, 3,4-position or 3,5-position. In trisubstituted phenyl groups, the substituents can be located in 2,3,4-position, 2,3,5-position, 2,3,6-position, 2,4,5-position, 2,4,6-position or 3,4,5-position.

Ring heteroatoms can be located in any positions, provided that the heterocyclic system is known in the art and is stable and suitable as a subgroup for the desired purpose of the compound of the formula I such as use as a drug substance. In one embodiment of the invention, two ring oxygen atoms cannot be present in adjacent ring positions of any heterocycle, in another embodiment two ring heteroatoms chosen from oxygen and sulfur cannot be present in adjacent ring positions of any heterocycle. Substituents on heterocyclic groups can be located in any positions. For example, in a pyridin-2-yl group substituents can be located in the 3-position and/or 4-position and/or 5-position and/or 6-position, in a pyridin-3-yl group substituent can be located in the 2-position and/or 4-position and/or 5-position and/or 6-position, in a pyridin-4-yl group substituents can be located in the 2-position and/or 3-position and/or 5-position and/or 6-position.

When an oxo group is bonded to a carbon atom, it replaces two hydrogen atoms on a carbon atom of the parent system. Thus, if a $CH_2$ group in a chain or a ring is substituted by oxo, i.e. by a doubly bonded oxygen atom, it becomes a CO group. Evidently, an oxo group cannot occur as a substituent on a carbon atom in an aromatic ring such as in a phenyl group, for example.

The present invention includes all stereoisomeric forms of the compounds of the formula I and their salts and solvates. With respect to each chiral center, independently of any other chiral center, the compounds of the formula I can be present in S configuration or substantially S configuration, or in R configuration or substantially R configuration, or as a mixture of the S isomer and the R isomer in any ratio. The invention includes all possible enantiomers and diastereomers and mixtures of two or more stereoisomers, for example mixtures of enantiomers and/or diastereomers, in all ratios. Thus, compounds according to the invention which can exist as enantiomers can be present in enantiomerically pure form, both as levorotatory and as dextrorotatory antipodes, and in the form of mixtures of the two enantiomers in all ratios including racemates. In the case of a E/Z isomerism, or cis/trans isomerism, for example on double bonds or rings such as cycloalkyl rings, the invention includes both the E form and Z form, or the cis form and the trans form, as well as mixtures of these forms in all ratios. In one embodiment of the invention, a compound which can occur in two or more stereoisomeric forms is a pure, or substantially pure, individual stereoisomer. The preparation of individual stereoisomers can be carried out, for example, by separation of a mixture of isomers by customary methods, for example by chromatography or crystallization, by the use of stereochemically uniform starting materials in the synthesis, or by stereoselective synthesis. Optionally, a derivatization can be carried out before a separation of stereoisomers. The separation of a mixture of stereoisomers can be carried out at the stage of the compound of the formula I or at the stage of a starting material or an intermediate during the synthesis. The present invention also includes all tautomeric forms of the compounds of the formula I and their salts and solvates.

In case the compounds of the formula I contain one or more acidic and/or basic groups, i.e. salt-forming groups, the invention also includes their corresponding physiologically or toxicologically acceptable salts, i.e. non-toxic salts, in particular their pharmaceutically acceptable salts.

The present invention furthermore includes all solvates of compounds of the formula I, for example hydrates or adducts with alcohols such as ($C_1$-$C_4$)-alkanols, active metabolites of the compounds of the formula I, and also prodrugs and derivatives of the compounds of the formula I which in vitro may not necessarily exhibit pharmacological activity but which in vivo are converted into pharmacologically active compounds, for example esters or amides of carboxylic acid groups.

Combinations

The compounds of the present invention can be widely combined with other pharmacologically active compounds, such as all drugs mentioned in the Rote Liste 2014, e.g. all antidiabetics mentioned in the Rote Liste 2014, chapter 12, all weight-reducing agents or appetite suppressants mentioned in the Rote Liste 2014, chapter 6, all lipid-lowering agents mentioned in the Rote Liste 2014, chapter 58, all antihypertensives mentioned in the Rote Liste 2014 chapter 17, all nephroprotectives mentioned in the Rote Liste, or all diuretics mentioned in the Rote Liste 2014, chapter 36. The active ingredient combinations can be applied either by separate administration of the active ingredients to the patient or in the form of combination products in which a plurality of active ingredients are present in one pharmaceutical preparation. When administered separately, administration may occur simultaneously or sequentially, in any order. The amount of the compound of the invention and the other pharmaceutically active ingredient(s) and the relative timings of administration will be selected in order to achieve the desired combined therapeutic effect. The administration of the combination may be concomitantly in: (1) a unitary pharmaceutical composition including all pharmaceutically active ingredients; or (2) separate pharmaceutical compositions each including at least one of the pharmaceutically active ingredients. Alternatively, the combination may be administered separately in a sequential manner wherein one treatment agent is administered first and the other second or vice versa. Such sequential administration may be close in time or remote in time.

Most of the active ingredients mentioned hereinafter are disclosed in the USP Dictionary of USAN and International Drug Names, US Pharmacopeia, Rockville 2012.

Therapeutic agents which are suitable for combinations include, for example, antidiabetic agents such as:

Insulin and insulin derivatives, for example: insulin glargine (e.g. Lantus®), insulin glulisine (e.g. Apidra®), insulin detemir (e.g. Levemir®), insulin lispro (e.g. Humalog®, Liprolog®), insulin degludec (e.g. DegludecPlus®, IdegLira (NN9068)), insulin aspart and aspart formulations (e.g. NovoLog®), basal insulin and analogues (e.g. LY2605541, LY2963016), PEGylated insulin lispro (e.g. LY-275585), long-acting insulins (e.g. NN1436, Insumera (PE0139), AB-101, AB-102, Sensulin LLC), intermediate-acting insulins (e.g. Humulin®N, Novolin®N), fast-acting and short-acting insulins (e.g. Humulin®R, Novolin®R, Linjeta®(VIAject®), PH20 insulin, NN1218, HinsBet®), premixed insulins, SuliXen®, NN1045, insulin plus Symlin®, ACP-002 insulin, and oral, inhalable, transdermal and buccal or sublingual insulins (e.g. Exubera®, Nasulin®, Afrezza®, insulin tregopil, TPM-02 Insulin, Capsulin®, Oral-lyn®, Cobalamin® oral insulin, ORMD-0801, Oshadi oral insulin, NN1953, NN1954, NN1956, VIAtab®).

Also suitable are those insulin derivatives which are bonded to albumin or another protein by a bifunctional linker.

Glucagon-like-peptide 1 (GLP-1), GLP-1 analogues, and GLP-1 receptor agonists, for example: lixisenatide (e.g. Lyxumia®), exenatide (e.g. exendin-4, rExendin-4, Byetta®, Bydureon®, exenatide NexP), liraglutide (e.g. Victoza®), semaglutide, taspoglutide, albiglutide, dulaglutide, ACP-003, CJC-1134-PC, GSK-2374697, PB-1023, TTP-054, langlenatide (HM-11260C), CM-3, AB-201, ORMD-0901, NN9924, NN9926, NN9927, Nodexen, Viador-GLP-1, CVX-096, ZYOG-1, ZYD-1, ZP-3022, CAM-2036, DA-3091, DA-15864, ARI-2651, ARI-2255, exenatide-XTEN (VRS-859), exenatide-XTEN + Glucagon-XTEN (VRS-859 + AMX-808) and polymer-bound GLP-1 and GLP-1 analogues.

Dual GLP-1/GIP agonists (e.g. RG-7697 (MAR-701), MAR-709, BHM081, BHM089, BHM098).
Dual GLP-1/glucagon receptor agonists (e.g. BHM-034, OAP-189 (PF-05212389, TKS-1225), TT-401/402, ZP2929, LAPS-HMOXM25, MOD-6030).
Dual GLP-1/gastrin agonists (e.g. ZP-3022).

Other suitable combination partners are:

Further gastrointestinal peptides such as peptide YY 3-36 (PYY3-36) or analogues thereof and pancreatic polypeptide (PP) or analogues thereof.

Glucagon receptor agonists or antagonists, glucose-dependent insulinotropic polypeptide (GIP) receptor agonists or antagonists, ghrelin antagonists or inverse agonists, xenin and analogues thereof.

Dipeptidyl peptidase-IV (DPP-4) inhibitors, for example: alogliptin (e.g. Nesina®, Kazano®), linagliptin (e.g. Ondero®, Trajenta®, Tradjenta®, Trayenta®), saxagliptin (e.g. Onglyza®, Komboglyze XR®), sitagliptin (e.g. Januvia®, Xelevia®, Tesavel®, Janumet®, Velmetia®, Juvisync®, Janumet XR®), anagliptin, teneligliptin (e.g. Tenelia®), trelagliptin, vildagliptin (e.g. Galvus®, Galvumet®), gemigliptin, omarigliptin, evogliptin, dutogliptin, DA-1229, MK-3102, KM-223, KRP-104, PBL-1427, Pinoxacin hydrochloride, and Ari-2243.

Sodium-dependent glucose transporter 2 (SGLT-2) inhibitors, for example: canagliflozin, dapagliflozin, remogliflozin, remogliflozin etabonate, sergliflozin, empagliflozin, ipragliflozin, tofogliflozin (RO-4998452), luseogliflozin, ertugliflozin (PF-04971729), EGT-0001442, LIK-066, SBM-TFC-039, and KGA-3235 (DSP-3235).
Dual inhibitors of SGLT-2 and SGLT-1 (e.g. LX-4211, LIK066).
SGLT-1 inhibitors (e.g. LX-2761, KGA-3235) or SGLT-1 inhibitors in combination with anti-obesity drugs such as ileal bile acid transfer (IBAT) inhibitors (e.g. GSK-1614235 + GSK-2330672).

Biguanides (e.g. metformin, buformin, phenformin).

Thiazolidinediones (e.g. pioglitazone, rivoglitazone, rosiglitazone, troglitazone), glitazone analogues (e.g. lobeglitazone).

Peroxisome proliferator-activated receptors (PPAR-)(alpha, gamma or alpha/gamma) agonists or modulators (e.g. aleglitazar, muraglitazar, tesaglitazar, saroglitazar (e.g. Lipaglyn®), GFT-505).

Sulfonylureas (e.g. tolbutamide, glibenclamide, glimepiride, Amaryl®, glipizide) and meglitinides (e.g. nateglinide, repaglinide, mitiglinide).

Alpha-glucosidase inhibitors (e.g. acarbose, miglitol, voglibose).

Amylin and amylin analogues (e.g. pramlintide, Symlin®).

G-protein coupled receptor 119 (GPR119) agonists (e.g. GSK-1292263, PSN-821, MBX-2982, APD-597, ARRY-981, ZYG-19, DS-8500, HM-47000, YH-Chem1).

GPR40 agonists (e.g. fasiglifam (TAK-875), TUG-424, P-1736, P-11187, JTT-851, GW9508, CNX-011-67, AM-1638, AM-5262).

GPR120 agonists and GPR142 agonists.

Systemic or low-absorbable TGR5 (GPBAR1 = G-protein-coupled bile acid receptor 1) agonists (e.g. INT-777, XL-475, SB756050).

Other suitable combination partners are:

Diabetes immunotherapeutics, for example: oral C-C chemokine receptor type 2 (CCR-2) antagonists (e.g. CCX-140), interleukin 1 beta (IL-1ß) antagonists (e.g. AC-201), or oral monoclonal antibodies (MoA) (e.g. methalozamide, VVP808, PAZ-320, P-1736, PF-05175157, PF-04937319).

Anti-inflammatory agents for the treatment of the metabolic syndrome and diabetes, for example: nuclear factor kappa B inhibitors (e.g. Triolex®).

Adenosine monophosphate-activated protein kinase (AMPK) stimulants, for example: Imeglimin (PXL-008), Debio-0930 (MT-63-78), R-481.

Inhibitors of 11-beta-hydroxysteroid dehydrogenase 1 (11-beta-HSD-1) (e.g. LY2523199, BMS770767, RG-4929, BMS816336, AZD-8329, HSD-016, BI-135585).

Activators of glucokinase (e.g. PF-04991532, TTP-399 (GK1-399), GKM-001 (ADV-1002401), ARRY-403 (AMG-151), TAK-329, TMG-123, ZYGK1).

Inhibitors of diacylglycerol O-acyltransferase (DGAT) (e.g. pradigastat (LCQ-908)), inhibitors of protein tyrosine phosphatase 1 (e.g. trodusquemine), inhibitors of glucose-6-phosphatase, inhibitors of fructose-1,6-bisphosphatase, inhibitors of glycogen phosphorylase, inhibitors of phosphoenol pyruvate carboxykinase, inhibitors of glycogen synthase kinase, inhibitors of pyruvate dehydrogenase kinase.

Modulators of glucose transporter-4, somatostatin receptor 3 agonists (e.g. MK-4256).

One or more lipid lowering agents are also suitable as combination partners, for example: 3-hydroxy-3-methylglutaryl-coenzym-A-reductase (HMG-CoA-reductase) inhibitors such as simvastatin (e.g. Zocor®, Inegy®, Simcor®), atorvastatin (e.g. Sortis®, Caduet®), rosuvastatin (e.g. Crestor®), pravastatin (e.g. Lipostat®, Selipran®), fluvastatin (e.g. Lescol®), pitavastatin (e.g. Livazo®, Livalo®), lovastatin (e.g. Mevacor®, Advicor®), mevastatin (e.g. Compactin®), rivastatin, cerivastatin (Lipobay®), fibrates such as bezafibrate (e.g. Cedur® retard), ciprofibrate (e.g. Hyperlipen®), fenofibrate (e.g. Antara®, Lipofen®, Lipanthyl®), gemfibrozil (e.g. Lopid®, Gevilon®), etofibrate, simfibrate, ronifibrate, clinofibrate, clofibride, nicotinic acid and derivatives thereof (e.g. niacin, including slow release formulations of niacin), nicotinic acid receptor 1 agonists (e.g. GSK-256073), PPAR-delta agonists, acetyl-CoA-acetyltransferase (ACAT) inhibitors (e.g. avasimibe), cholesterol absorption inhibitors (e.g. ezetimibe, Ezetrol®, Zetia®, Liptruzet®, Vytorin®, S-556971), bile acid-binding substances (e.g. cholestyramine, colesevelam), ileal bile acid transport (IBAT) inhibitors (e.g. GSK-2330672, LUM-002), microsomal triglyceride transfer protein (MTP) inhibitors (e.g. lomitapide (AEGR-733), SLx-4090, granotapide), modulators of proprotein convertase subtilisin/kexin type 9 (PCSK9) (e.g. alirocumab (REGN727/SAR236553), AMG-145, LGT-209, PF-04950615, MPSK3169A, LY3015014, ALD-306, ALN-PCS, BMS-962476, SPC5001, ISIS-394814, 1B20, LGT-210, 1D05, BMS-PCSK9Rx-2, SX-PCK9, RG7652), LDL receptor up-regulators, for example liver selective thyroid hormone receptor beta agonists (e.g. eprotirome (KB-2115), MB07811, sobetirome (QRX-431), VIA-3196, ZYT1), HDL-raising compounds such as: cholesteryl ester transfer protein (CETP) inhibitors (e.g. torcetrapib, anacetrapib (MK0859), dalcetrapib, evacetrapib, JTT-302, DRL-17822, TA-8995, R-1658, LY-2484595, DS-1442), ATP-binding cassette (ABC1) regulators, lipid metabolism modulators (e.g. BMS-823778, TAP-301, DRL-21994, DRL-21995), phospholipase A2 (PLA2) inhibitors (e.g. darapladib, Tyrisa®, varespladib, rilapladib), ApoA-I enhancers (e.g. RVX-208, CER-001, MDCO-216, CSL-112), cholesterol synthesis inhibitors (e.g. ETC-1002), lipid metabolism modulators (e.g. BMS-823778, TAP-301, DRL-21994, DRL-21995) and omega-3 fatty acids and derivatives thereof (e.g. icosapent ethyl (AMR101), Epanova®, AKR-063, NKPL-66, PRC-4016, CAT-2003).

Other suitable combination partners are one or more active substances for the treatment of obesity, such as for example:

Bromocriptine (e.g. Cycloset®, Parlodel®), phentermine and phentermine formulations or combinations (e.g. Adipex-P, Ionamin, Qsymia®), benzphetamine (e.g. Didrex®), diethylpropion (e.g. Tenuate®), phendimetrazin (e.g. Adipost®, Bontril®), bupropion and combinations (e.g. Zyban®, Wellbutrin XL®, Contrave®, Empatic®), sibutramine (e.g. Reductil®, Meridia®), topiramat (e.g. Topamax®), zonisamid (e.g. Zonegran®), tesofensine, opioid antagonists such as naltrexone (e.g. Naltrexin®, naltrexone + bupropion), cannabinoid receptor 1 (CB1) antagonists (e.g. TM-38837), melanin-concentrating hormone (MCH-1) antagonists (e.g. BMS-830216, ALB-127158(a)), MC4 receptor agonists and partial agonists (e.g. AZD-2820, RM-493), neuropeptide Y5 (NPY5) or NPY2 antagonists (e.g. velneperit, S-234462), NPY4 agonists (e.g. PP-1420), beta-3-adrenergic receptor agonists, leptin or leptin mimetics, agonists of the 5-hydroxytryptamine 2c (5HT2c) receptor (e.g. lorcaserin, Belviq®), pramlintide/metreleptin, lipase inhibitors such as cetilistat (e.g. Cametor®), orlistat (e.g. Xenical®, Calobalin®), angiogenesis inhibitors (e.g. ALS-L1023), betahistidin and histamine H3 antagonists (e.g. HPP-404), AgRP (agouti related protein) inhibitors (e.g. TTP-435), serotonin re-uptake inhibitors such as fluoxetine (e.g. Fluctine®), duloxetine (e.g. Cymbalta®), dual or triple monoamine uptake inhibitors (dopamine, norepinephrine and serotonin re-uptake) such as sertraline (e.g. Zoloft®), tesofensine, methionine aminopeptidase 2 (MetAP2) inhibitors (e.g. beloranib), and antisense oligonucleotides against production of fibroblast growth factor receptor 4 (FGFR4) (e.g. ISIS-FGFR4Rx) or prohibitin targeting peptide-1 (e.g. Adipotide®).

Moreover, combinations with drugs for influencing high blood pressure, chronic heart failure or atherosclerosis, for example: nitric oxide donors, AT1 antagonists or angiotensin II (AT2) receptor antagonists such as telmisartan (e.g. Kinzal®, Micardis®), candesartan (e.g. Atacand®, Blopress®), valsartan (e.g. Diovan®, Co-Diovan®), losartan (e.g. Cosaar®), eprosartan (e.g. Teveten®), irbesartan (e.g. Aprovel®, CoAprovel®), olmesartan (e.g. Votum®, Olmetec®), tasosartan, azilsartan (e.g. Edarbi®), dual angiotensin receptor blockers (dual ARBs), angiotensin converting enzyme (ACE) inhibitors, ACE-2 activators, renin inhibitors, prorenin inhibitors, endothelin converting enzyme (ECE) inhibitors, endothelin receptor (ET1/ETA) blockers, endothelin antagonists, diuretics, aldosterone antagonists, aldosterone synthase inhibitors, alpha-blockers, antagonists of the alpha-2 adrenergic receptor, beta-blockers, mixed alpha-/beta-blockers, calcium antagonists, calcium channel blockers (CCBs), nasal formulations of the calcium channel blocker diltiazem (e.g. CP-404), dual mineralocorticoid/CCBs, centrally acting antihypertensives, inhibitors of neutral endopeptidase, aminopeptidase-A inhibitors, vasopeptide inhibitors, dual vasopeptide inhibitors such as neprilysin-ACE inhibitors or neprilysin-ECE inhibitors, dual-acting AT receptor-neprilysin inhibitors, dual AT1/ETA antagonists, advanced glycation end-product (AGE) breakers, recombinant renalase, blood pressure vaccines such as anti-RAAS (renin-angiotensin-aldosteron-system) vaccines, AT1- or AT2-vaccines, drugs based on hypertension pharmacogenomics such as modulators of genetic polymorphisms with antihypertensive response, thrombocyte aggregation inhibitors, and others or combinations thereof are suitable.

In another aspect, this invention relates to the use of a compound according to the invention or a physiologically acceptable salt thereof combined with at least one of the active substances described above as a combination partner, for preparing a medicament which is suitable for the treatment or prevention of diseases or conditions which can be affected by binding to the GPR119 and modulating its activity. This is preferably a disease in the context of the metabolic syndrome, particularly one of the diseases or conditions listed above, most particularly diabetes or obesity or complications thereof.

The use of the compounds according to the invention, or a physiologically acceptable salt thereof, in combination with one or more active substances may take place simultaneously, separately or sequentially.

The use of the compound according to the invention, or a physiologically acceptable salt thereof, in combination with another active substance may take place simultaneously or at staggered times, but particularly within a short space of time. If they are administered simultaneously, the two active substances are given to the patient together; if they are used at staggered times, the two active substances are given to the patient within a period of less than or equal to 12 hours, but particularly less than or equal to 6 hours.

Consequently, in another aspect, this invention relates to a medicament which comprises compounds according to the invention or a physiologically acceptable salt of such a compound and at least one of the active substances described above as combination partners, optionally together with one or more inert carriers and/or diluents.

The compounds according to the invention, or physiologically acceptable salt or solvate thereof, and the additional active substance to be combined therewith may both be present together in one formulation, for example a tablet or capsule, or separately in two identical or different formulations, for example as so-called kit-of-parts.

Compounds according to the invention can be administered to animals, in particular to mammals including humans, as pharmaceuticals by themselves, in mixtures with one another, or in the form of pharmaceutical compositions. The administration can be carried out orally, for example in the form of tablets, film-coated tablets, sugar-coated tablets, granules, hard and soft gelatin capsules, solutions including aqueous, alcoholic and oily solutions, juices, drops, syrups, emulsions or suspensions, rectally, for example in the form of suppositories, or parenterally, for example in the form of solutions for subcutaneous, intramuscular or intravenous injection or infusion, in particular aqueous solutions.

Suitable pharmaceutical compositions for oral administration may be in the form of separate units, for example capsules, cachets, lozenges or tablets, each of which contains a defined amount of the compound of formula I; as powders or granules; as solution or suspension in an aqueous or nonaqueous liquid; or as an oil-in-water or water-in-oil emulsion. These compositions may, as already mentioned, be prepared by any suitable pharmaceutical method which includes a step in which the active ingredient and the carrier (which may consist of one or more additional ingredients) are brought into contact. The compositions are generally produced by uniform and homogeneous mixing of the active ingredient with a liquid and/or finely divided solid carrier, after which the product is shaped if necessary. Thus, for example, a tablet can be produced by compressing or molding a powder or granules of the compound, where appropriate with one or more additional ingredients. Compressed tablets can be produced by tableting the compound in free-flowing form such as, for example, a powder or granules, where appropriate mixed with a binder, glidant, inert diluent and/or one (or more) surfactant(s)/dispersant(s) in a suitable machine. Molded tablets can be produced by molding the compound, which is in powder form and has been moistened with an inert liquid diluent, in a suitable machine.

Pharmaceutical compositions which are suitable for peroral (sublingual) administration comprise lozenges which contain a compound of formula I with a flavoring, typically sucrose, and gum arabic or tragacanth, and pastilles which comprise the compound in an inert base such as gelatin and glycerol or sucrose and gum arabic.

Coated formulations and coated slow-release formulations, especially acid- and gastric juice-resistant formulations, also belong within the framework of the invention. Suitable coatings resistant to gastric juice comprise cellulose acetate phthalate, polyvinyl acetate phthalate, hydroxypropylmethylcellulose phthalate and anionic polymers of methacrylic acid and methyl methacrylate.

Pharmaceutical compositions suitable for rectal administration are preferably in the form of single-dose suppositories. These can be produced by mixing a compound of formula I with one or more conventional solid carriers, for example cocoa butter, and shaping the resulting mixture.

Pharmaceutical compositions suitable for parenteral administration comprise preferably sterile aqueous preparations of a compound of formula I, which are preferably isotonic with the blood of the intended recipient. These preparations are preferably administered intravenously, although administration may also take place by subcutaneous, intramuscular or intradermal injection. These preparations can preferably be produced by mixing the compound with water and making the resulting solution sterile and isotonic with blood. Injectable compositions of the invention generally contain 0.1 to 5% by weight of the active compound.

Other suitable administration forms are, for example, percutaneous or topical administration, for example in the form of ointments, creams, tinctures, sprays, powders or transdermal therapeutic systems, or inhalative administration, for example in the form of nasal sprays or aerosol mixtures, or forms such as microcapsules, implants or rods.

Pharmaceutical compositions suitable for topical use on the skin are preferably in the form of ointment, cream, lotion, paste, spray, aerosol or oil. The carriers used may be petrolatum, lanolin, polyethylene glycols, alcohols and combinations of two or more of these substances. The active ingredient is generally present in a concentration of 0.1 to 15% by weight of the composition, for example 0.5 to 2%.

Transdermal administration is also possible. Pharmaceutical compositions suitable for transdermal uses may be in the form of single patches which are suitable for long-term close contact with the patient's epidermis. Such patches suitably contain the active ingredient in an aqueous solution which is buffered where appropriate, dissolved and/or dispersed in an adhesive or dispersed in a polymer. A suitable active ingredient concentration is about 1% to 35%, preferably about 3% to 15%. A particular option is for the active ingredient to be released by electrotransport or iontophoresis as described, for example, in Pharmaceutical Research, 2(6): 318 (1986).

Compounds according to the invention can additionally be used in systems for local drug delivery, for example in coated stents for preventing or reducing in-stent restenosis or by applying them locally by means of a catheter. The appropriate administration form depends, among others, on the disease to be treated and on its severity.

The dosing of compounds according to the invention to achieve the desirable therapeutic effect depends on a number of factors, for example the specific compound chosen, the intended use, the mode of administration and the clinical condition of the patient. The daily dose is generally in the range from 0.3 mg to 100 mg (typically from 3 mg to 50 mg) per day and per kilogram of body weight, for example 3-10 mg/kg/day. An intravenous dose may be, for example, in the range from 0.3 mg to 1.0 mg/kg, which can suitably be administered as infusion of 10 ng to 100 ng per kilogram and per minute. Suitable infusion solutions for these purposes may contain, for example, 0.1 ng to 100 mg, typically 1 ng to 100 mg, per milliliter. Single doses may contain, for example, 1 mg to 10 g of the active ingredient. Thus, ampoules for injections may contain, for example, from 1 mg to 100 mg, and orally administrable single-dose formulations, for example tablets or capsules, may contain, for example, from 1.0 to 1000 mg, typically from 10 to 600 mg. For prevention and/or treatment of the abovementioned conditions, the compounds of the formula I themselves may be used as the compound, but they are preferably present with a compatible carrier in the form of a pharmaceutical composition. The carrier must, of course, be acceptable in the sense that it is compatible with the other ingredients of the composition and is not harmful for the patient's health. The carrier may be a solid or a liquid or both and is preferably formulated with the compound as a single dose, for example as a tablet, which may contain 0.05% to 95% by weight of the active ingredient. Other pharmaceutically active substances may likewise be present, including other compounds of formula I. The pharmaceutical compositions of the invention can be produced by one of the known pharmaceutical methods, which essentially consist of mixing the ingredients with pharmacologically acceptable carriers and/or excipients.

Another subject of the present invention are processes for the preparation of the compounds of the formula I and their salts and solvates, by which the compounds are obtainable and which are outlined in the following.

Abbreviations

Abbreviations within this document have their common meanings unless defined otherwise herein. An exemplary list of abbreviations used, can be found below.

| | |
|---|---|
| Ac | acetyl |
| amu | atomic mass unit |
| atm | atmosphere (pressure unit, 101325 Pa) |
| BSA | bovine serum albumin |
| cAMP | cyclic adenosine monophosphate |
| cat. | catalyst / catalyzed |
| CDI | carbonyl diimidazole |
| dba | dibenzylideneacetone |
| DCM | dichloromethane |
| DEAD | diethyl azodicarboxylate |
| DIAD | diisopropyl azodicarboxylate |

(continued)

| | |
|---|---|
| DIPEA | diisopropyl-ethyl-amine |
| DMEM | Dulbecco's modified eagle medium |
| DMF | dimethylformamide |
| DMSO | dimethylsulfoxide |
| dppf | diphenylphosphinoferrocene |
| EA | ethyl acetate |
| $EC_{50}$ | concentration causing 50% of the maximal response |
| EDC | ethyl dimethylaminopropyl carbodiimide |
| ESI | electrospray ionisation |
| FA | formic acid |
| FCS | fetal calf serum |
| h | hour(s) |
| Hal | halogen (atom) |
| HATU | *O*-(7-azabenzotriazol-1-yl)-*N*,*N*,*N'*,*N'*-tetramethyl-uronium hexafluorophosphate |
| HBSS | Hank's buffered salt solution |
| HEK 293 | human embryonic kidney 293 |
| HEPES | 4-(2-hydroxyethyl)-1-piperazineethanesulfonic acid |
| HOBt | 1-hydroxy-benzotriazole |
| HPLC | high pressure liquid chromatography |
| HTRF | homogenous time-resolved fluorescence |
| IC50 | half maximal inhibitory concentration |
| LCMS | liquid chromatography coupled mass spectroscopy |
| LG | leaving group |
| MeCN | methylcyanide (acetonitrile) |
| min | minute(s) |
| MS | mass spectroscopy |
| MTBE | methyl tert.-butyl ether |
| NMP | *N*-methyl pyrrolidin-2-one |
| NMR | nuclear magnetic resonance (spectrum) |
| PBS | phosphate buffered saline |
| PE | petroleum ether |
| PMBCI | para-methoxybenzyl chloride |
| Rt | retention time |
| r.t. | room temperature |
| SGC | silica gel chromatography |
| $SiO_2$ | silica gel (for chromatography) |
| TFA | trifluoroacetic acid |
| THF | tetrahydrofuran |
| TM | transition metal |

(continued)

| | |
|---|---|
| TMS | tetramethylsilane |
| Ts | para-tolylsulfonyl |
| UV | ultraviolet (spectrum) |

Synthetic Methods

Detailed descriptions of the Typical Procedures to which reference is made in this section can be found in the Examples section.

Analytical Methods

The prepared compounds were in general characterized by spectroscopic data and chromatographic data, in particular mass spectra (MS) and HPLC retention times (Rt; in min) which were obtained by combined analytical HPLC/MS characterization (LC/MS), In the MS characterization, in general the mass number (m/z) of the peak of the molecular ion [M], for example [$M^+$], or of a related ion such as the ion [M+1], for example [$(M+1)^+$], i.e. the protonated molecular ion [$(M+H)^+$] ([$MH^+$]), or the ion [M-1], for example [$(M-1)^-$], i.e. the deprotonated molecular ion [$(M-H)^-$], which was formed depending on the ionization method used, is given.

The particulars of the LC/MS methods used are as follows. "ACN" means acetonitrile, "TFA" means trifluoroacetic acid, and "FA" means formic acid. Unless specified otherwise, the MS ionization method was electrospray ionization ES+. LC/MS Method A: Column: Waters UPLC BEH C18 2,1*50 mm; 1,7 μm; eluent A: water + 0.05 % FA; eluent B: ACN + 0.035 % FA; gradient: 95 A : 5 % B (0 min) to 5 % A : 95 % B (2.6 min) to 95 % A : 5 % B (2.7 min) to 95 % A : 5 % B (3 min); flow: 0.9 ml/min at 55°C

Pharmacological tests

Keap1-Nrf2 peptide binding assay

A fluorescence polarisation assay was used to measure binding of the human Keap1 kelch domain (amino acids Ala321-Thr609) to a fluorescently labeled peptide containing a Keap1 binding site of human Nrf2 (Alexa633-AFFAQLQLDEETGEFL; JPT Technologies, Berlin, Germany). Two microliters of test compound in reaction buffer (phosphate-buffered saline, 2 mM DL-dithiothreitol, 0.015 % (v/v) Brij-35) containing 6 % (v/v) DMSO were mixed with two microliters of the Keap1 kelch domain in reaction buffer in a black 384-well small volume microtiter plate and incubated at ambient temperature for 15 minutes. Thereafter, two microliters were added of the fluorescently labeled Nrf2 peptide in reaction buffer, and the mixture was incubated for a further 30 minutes at ambient temperature. Final concentrations of the Keap1 kelch domain and the fluorescently labeled Nrf2 peptide were 20 nM and 50 nM, respectively.

Fluorescence polarization (FP) was measured at excitation and emission wavelengths of 635 nm and 660 nm, respectively. Positive controls for binding (two microliters of reaction buffer containing 6 % (v/v) DMSO instead of test compound in the same buffer) and negative controls (as positive controls, but without Keap1 kelch domain) were included on each microtiter plate. Data were expressed as percent-inhibition values:

$$\text{percent inhibition} = 100 \cdot \left(1 - \frac{\left|\mathrm{FP}_{\text{compound}} - \mathrm{FP}_{\text{negative control}}\right|}{\left|\mathrm{FP}_{\text{positive control}} - \mathrm{FP}_{\text{negative control}}\right|}\right)$$

$IC_{50}$ values were determined by testing different compound concentrations and fitting the resulting percent-inhibition values to the following equation using XLFit4:

$$\text{percent inhibition} = A + \frac{B - A}{1 + \left(\frac{IC_{50}}{[Compound]}\right)^{D}}$$

Parameter A was set to zero, pre-fit values for parameters B and D were set to 100 and 1, respectively.

Reporter gene assay

Into the pGL3 Luciferase Reporter Vector (Promega, Mannheim, Germany) containing the neomycin-resistance cassette, an oligonucleotide with the sequence eight repeats of the ARE (5'-GTGACAAAGCA-3') was cloned upstream of the SV40 promoter. L6 rat muscle cells were transfected with this construct and selected for neomycin resistance. The clone was propagated based on the ability of described Nrf2 activators such as sulforaphane to induce reporter-gene expression. In order to test for cellular activity, the cell clone was tested by incubating the cells for 16 - 20 h with the compounds. Reporter-gene assays were performed using the Bright Glo luciferase assay system (Promega). Nrf2-dependent regulation of the reporter gene was demonstrated using RNA interference. The cell clone was transfected in reverse mode with either non-silencing RNA or with two different small interfering RNAs for Nrf2, Sigma #SASI_Hs01_00182393 and Qiagen #SI03246950 using Lipofectamine RNAiMax (Invitrogen) according to the manufacturer's instructions. Subsequently, cells were plated at a concentration of 15,000 cells per well in collagen-treated 96-well plates. 24 h after seeding, the test compounds were added to the wells. Analysis of luciferase activity and gene expression were performed after a subsequent incubation for 20 h.

The EC500 value was defined as the compound concentration that increased the luciferase activity 5 times in the absence of a compound. The value was calculated by incubating the cells with different compound concentrations and fitting the luciferase activity using XLFit4.

Examples

The following examples are particular embodiments of the invention. They partially illustrate the scope of the invention without limiting it.

Abbreviations and chemical symbols have their usual and customary meanings unless otherwise indicated. The examples were prepared, isolated and analyzed by the procedures and methods given. Alternatively they may be prepared by the general synthetic methods known from the literature. Further variations of the synthetic procedures may be proposed by a person skilled in the art.
When example compounds containing a basic group were purified by preparative HPLC on reversed phase column material and, as customary, the eluent was a gradient mixture of water and acetonitrile containing trifluoroacetic acid (TFA), they were obtained in part in the form of their addition salt with TFA, depending on the details of the workup such as evaporation or lyophilization conditions. In the names of the example compounds and their structural formulae any such TFA present is not specified.

Example 1:

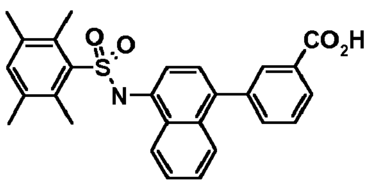

3-(4-(2,3,5,6-tetramethylphenylsulfonamido)naphthalen-1-yl)benzoic acid

1a) N-(4-bromonaphthalen-1-yl)-2,3,5,6-tetramethylbenzenesulfonamide

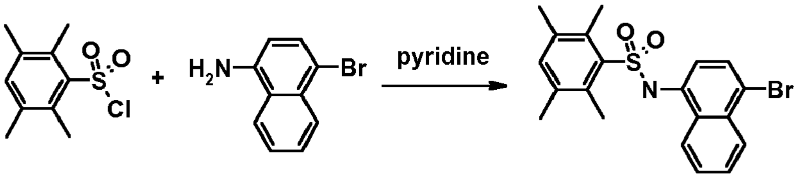

To a solution of 4-bromonaphthalen-1-amine (500 mg, 2.25 mmol) in dichloromethane (5 mL) were added pyridine (220 μL, 2.7 mmol) and 2,3,5,6-tetramethylbenzene-1-sulfonyl chloride (576 mg, 2.5 mmol). The reaction mixture was stirred at room temperature for 18 h and then treated with $NaHCO_3$ (5 mL, saturated aqueous solution). After stirring for 20 min at room temperature the reaction mixture was extracted with dichloromethane (20 mL). The organic phase was washed with HCl (aq., 2 M), dried over magnesium sulfate, filtered and then concentrated in vacuo. The title compound was obtained as white solid (838 mg, 89%) which was used in the next step 1b) without further purification.

1 b) tert-butyl 3-(4-(2,3,5,6-tetramethylphenylsulfonamido)naphthalen-1-yl)benzoate

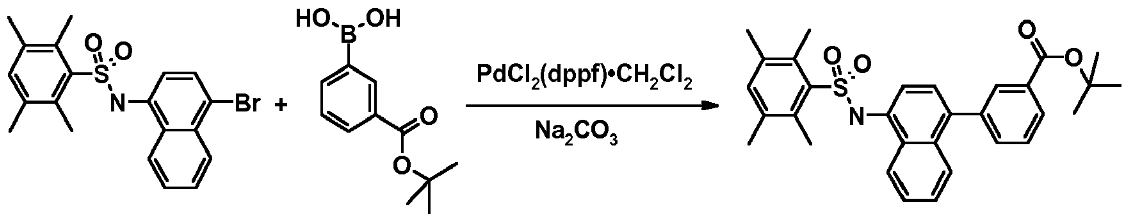


To a solution of N-(4-bromonaphthalen-1-yl)-2,3,5,6-tetramethylbenzene-sulfonamide (75 mg, 0.18 mmol) in dioxane (1.6 mL) were added (3-(tert-butoxycarbonyl)phenyl)-boronic acid (48 mg, 0.22 mmol), $PdCl_2(dppf)•CH_2Cl_2$ complex (8 mg, 0.009 mmol) and sodium carbonate (0.4 mL, 1mol/l aqueous solution). The reaction mixture was stirred at 85°C for 5 h. After cooling to room temperature, the reaction mixture was diluted with dichloromethane (10 mL), and washed with $NH_4Cl$ (saturated aqueous solution). The organic phase was dried over sodium sulfate, filtered and then concentrated in vacuo. The resulting residue was purified via silica gel flash chromatography with n-heptane / ethyl acetate (2% to 30%) as eluent to yield 40 mg (43 %) of the title compound as a white powder.

1c) 3-(4-(2,3,5,6-tetramethylphenylsulfonamido)naphthalen-1-yl)benzoic acid

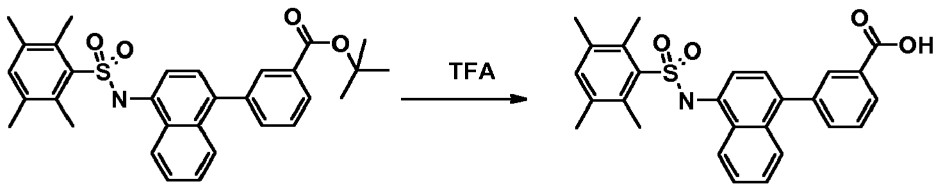


To a solution of tert-butyl 3-(4-(2,3,5,6-tetramethylphenylsulfonamido)naphthalen-1-yl)benzoate (30 mg, 0.058 mmol) in dichloromethane (2 mL) was added trifluoroacetic acid (200 μl, 2.6 mmol). The reaction mixture was stirred at room temperature for 18 h and then washed with $H_2O$ (3 mL), dried over magnesium sulfate, filtered and then concentrated in vacuo to yield 19 mg (71%) of the title compound.

Example 2:

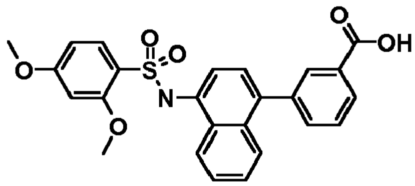

3-(4-(2,4-dimethoxyphenylsulfonamido)naphthalen-1-yl)benzoic acid

2a) tert-butyl 3-(4-aminonaphthalen-1-yl)benzoate

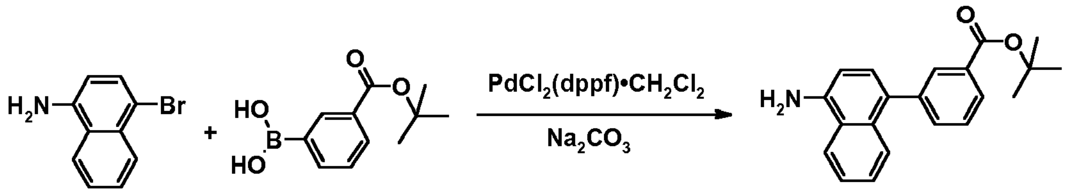


To a solution of 4-bromonaphthalen-1-amine (100 mg, 0.45 mmol) in dioxane (20 mL) were added (3-(tert-butoxycarbonyl)phenyl)boronic acid (120 mg, 0.54 mmol), $PdCl_2$(dppf)•$CH_2Cl_2$ complex (19 mg, 0.023 mmol) and sodium carbonate (0.9 mL, 1mol/l aqueous solution). The reaction mixture was stirred at 90°C for 4 h. After cooling to room temperature, dichloromethane (10 mL) and $H_2O$ were added. The organic phase was separated and washed with $H_2O$, dried over sodium sulfate, filtered and concentrated in vacuo to afford 150 mg (quantitative yield) of the title compound, which was used in the next step 2b) without further purification.

2b) 3-(4-(2,4-dimethoxyphenylsulfonamido)naphthalen-1-yl)benzoic acid

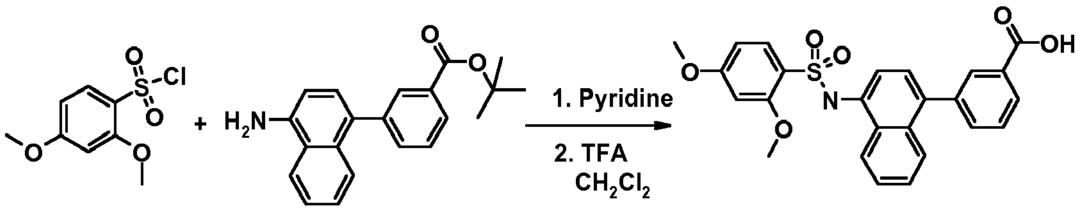


To a solution of tert-butyl 3-(4-aminonaphthalen-1-yl)benzoate (70 mg, 0.22 mmol) in dichloromethane (2 mL) were added pyridine (35 μL, 0.4 mmol) and 2,4-dimethoxy-benzenesulfonyl chloride (100 mg, 0.4 mmol). The reaction mixture was stirred at room temperature for 30 h. Afterwards the reaction mixture was diluted with dichloromethane (5 mL) and washed with hydrogen chloride (1 M aqueous solution), dried over magnesium sulfate, filtered and then concentrated in vacuo.
The resulting residue was dissolved in dichloromethane (3 mL). Trifluoroacetate (0.3 mL, 4 mmol) was added and the reaction mixture was stirred at room temperature for 3 h. The solvent was removed in vacuo and the resulting residue was purified by high performance liquid chromatography (RP silica gel, cetonitrile/water/trifluoroacetic acid). Lyophilization of the product fractions provided 48 mg, 47% of the title compound as a white powder.

Example 5:

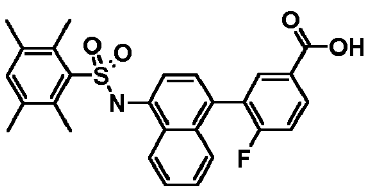

4-fluoro-3-(4-(2,3,5,6-tetramethylphenylsulfonamido)naphthalen-1-yl)benzoic acid

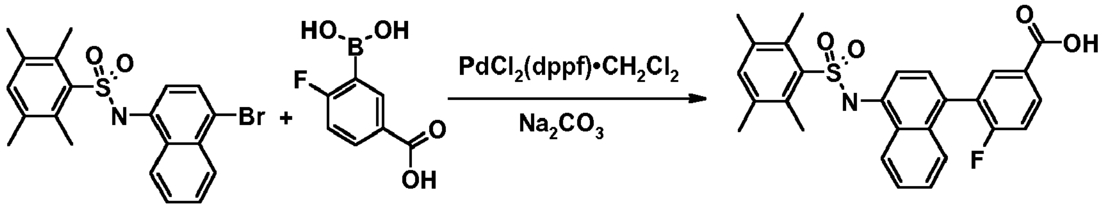


To a solution of N-(4-bromonaphthalen-1-yl)-2,3,5,6-tetramethylbenzene-sulfonamide (Example 1 a) (100 mg, 0.24 mmol) in dioxane (2 mL) were added 3-borono-4-fluorobenzoic acid (53 mg, 0.29 mmol), $PdCl_2$(dppf)•$CH_2Cl_2$ complex (10 mg, 0.012 mmol) and sodium carbonate (0.5 mL, 1mol/l aqueous solution). The reaction mixture was stirred at 85°C for 4 h. After cooling to room temperature, the reaction mixture was concentrated in vacuo. Purification of the residue by high performance liquid chromatography (RP silica gel, acetonitrile/water/trifluoroacetic acid) and lyophilization of the product fractions provided 20 mg (18 %) of the title compound as a white powder.

Example 6:

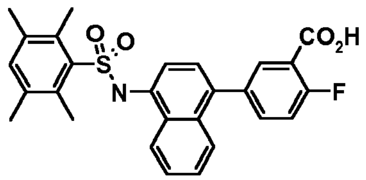

2-fluoro-5-(4-(2,3,5,6-tetramethylphenylsulfonamido)naphthalen-1-yl)benzoic acid

6a) methyl 2-fluoro-5-(4-(2,3,5,6-tetramethylphenylsulfonamido)naphthalen-1-yl)benzoate

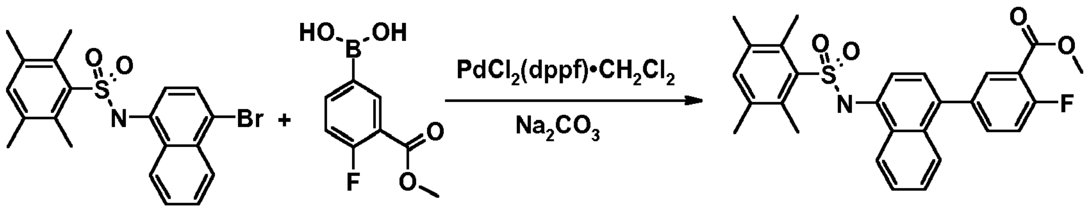


To a solution of N-(4-bromonaphthalen-1-yl)-2,3,5,6-tetramethylbenzene-sulfonamide (Example 1 a) (100 mg, 0.24 mmol) in dioxane (2 mL) were added (4-fluoro-3-(methoxycarbonyl)phenyl)boronic acid (57 mg, 0.29 mmol), $PdCl_2$(dppf)•$CH_2Cl_2$ complex (10 mg, 0.012 mmol) and sodium carbonate (0.5 mL, 1mol/l aqueous solution). The reaction mixture was stirred at 85°C for 1 h. After cooling to room temperature, the reaction mixture was then concentrated in vacuo. Purification of the residue by high performance liquid chromatography (RP silica gel, acetonitrile/water/trifluoroacetic acid) and lyophilization of the product fractions provided 91 mg (78 %) of the title compound as a white powder.

6b) 2-fluoro-5-(4-(2,3,5,6-tetramethylphenylsulfonamido)naphthalen-1-yl)benzoic acid

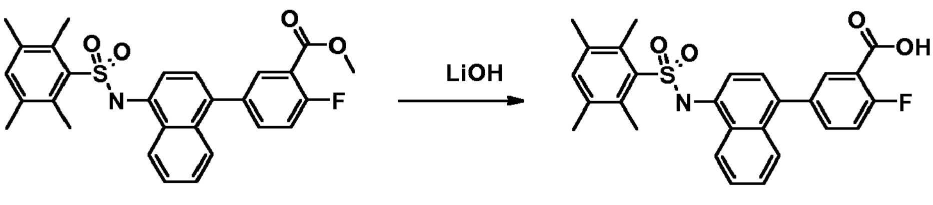

To a solution of methyl 2-fluoro-5-(4-(2,3,5,6-tetramethylphenylsulfonamido)-naphthalen-1-yl)benzoate (91 mg, 0.185 mmol) in a mixture of THF (2 mL), $H_2O$ (1.2 mL) and methanol (1 mL) was added lithium hydroxide (15 mg, 0.63 mmol). After stirring at room temperature for 1.5 h, the reaction mixture was diluted with THF (10 mL), neutralized with acetic acid, and concentrated in vacuo. Purification of the residue by high performance liquid chromatography (RP silica gel, acetonitrile/water/trifluoroacetic acid) and lyophilization of the product fractions provided 48 mg (54 %) of the title compound as a white powder.

Example 8:

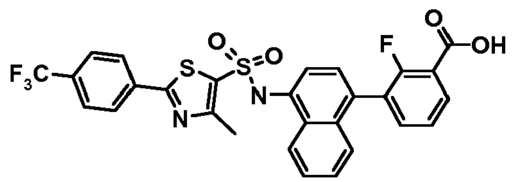

2-fluoro-3-(4-(4-methyl-2-(4-(trifluoromethyl)phenyl)thiazole-5-sulfonamido)-naphthalen-1-l)benzoic acid

8a) 3-(4-aminonaphthalen-1-yl)-2-fluorobenzoic acid

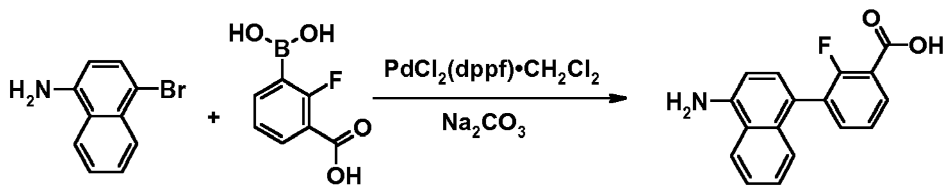


To a solution of 4-bromonaphthalen-1-amine (300 mg, 1.35 mmol) in dioxane (8 mL) were added 3-borono-2-fluorobenzoic acid (273 mg, 1.49 mmol), $PdCl_2$(dppf)•$CH_2Cl_2$ complex (58 mg, 0.068 mmol) and sodium carbonate (2.7 mL, 1mol/l aqueous solution). The reaction mixture was stirred at 90°C for 1 h. After cooling to room temperature, the reaction mixture was then concentrated in vacuo. Purification of the residue by high performance liquid chromatography (RP silica gel, acetonitrile/water/trifluoroacetic acid) and lyophilization of the product fractions provided 225 mg (59 %) of the title compound as a white powder.

8b) 2-fluoro-3-(4-(4-methyl-2-(4-(trifluoromethyl)phenyl)thiazole-5-sulfonamido)-naphthalen-1-l)benzoic acid

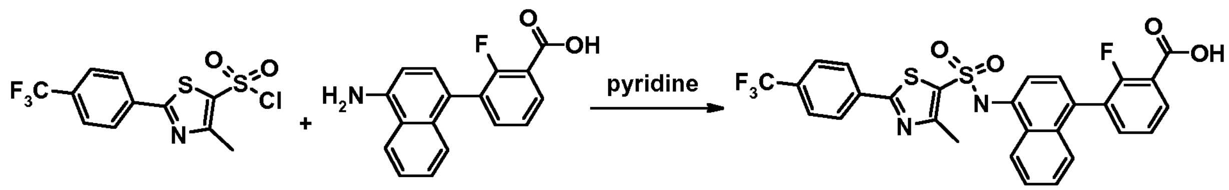


To a solution of 3-(4-aminonaphthalen-1-yl)-2-fluorobenzoic acid (75 mg, 0.267 mmol) in dichloromethane (3 mL) were added pyridine (25 mg, 26 µL, 0.32 mmol) and 4-methyl-2-(4-(trifluoromethyl)phenyl)thiazole-5-sulfonyl chloride (100 mg, 0.293 mmol). The reaction mixture was stirred at room temperature overnight. Afterwards the reaction mixture was washed with hydrogen chloride (1 M aqueous solution), dried over magnesium sulfate, filtered and then concentrated in vacuo. The resulting residue was purified by high performance liquid chromatography (RP silica gel, acetonitrile/water/trifluoroacetic acid). Lyophilization of the product fractions provided 15 mg (10 %) of the title compound as a white powder.

Example 59:

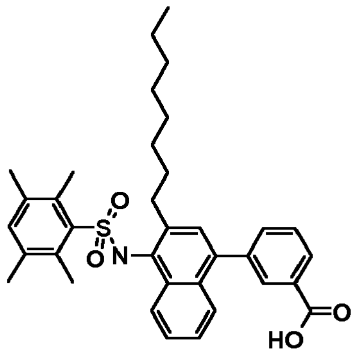

3-[3-octyl-4-[(2,3,5,6-tetramethylphenyl)sulfonylamino]-1-naphthyl]benzoic acid

59a) Methyl 3-(4-amino-3-bromo-1-naphthyl)benzoate

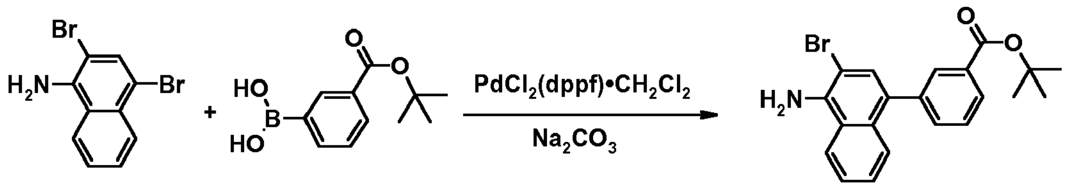


To a solution of 2,4-dibromonaphthalen-1-amine (100 mg, 0.3 mmol) in dioxane (2 mL) were added (3-(tert-butoxycarbonyl)phenyl)boronic acid (64 mg, 0.287 mmol), $PdCl_2$(dppf)·$CH_2Cl_2$ complex (12 mg, 0.014 mmol) and sodium carbonate (0.57 mL, 1mol/l aqueous solution). The reaction mixture was stirred at 90°C for 4 h. After cooling to room temperature, $H_2O$ (5 mL) was added, and the mixture was extracted with ethyl acetate. The combined organic phases were washed with $H_2O$, dried over sodium sulfate, filtered and then concentrated in vacuo. Purification of the residue by high performance liquid chromatography (RP silica gel, acetonitrile/water/ trifluoroacetic acid) and lyophilization of the product fractions provided 50 mg (44 %) of the title compound as a white powder.

59b) tert-butyl 3-(2-bromo-4-(2,3,5,6-tetramethylphenylsulfonamido)naphthalen-1-yl)benzoate

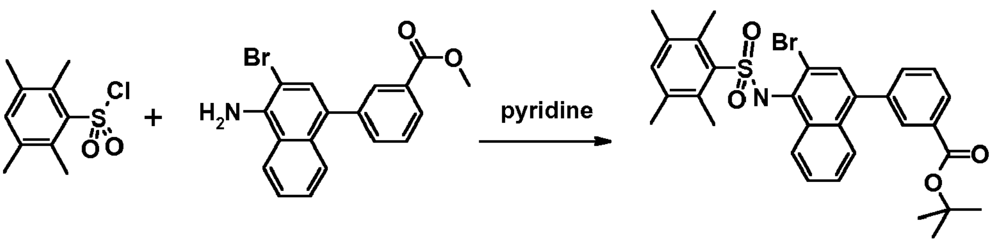


To a solution of methyl 3-(4-amino-3-bromo-1-naphthyl)benzoate (50 mg, 0.125 mmol) in dichloromethane (1 mL) were added pyridine (12 μL, 0.15 mmol) and 2,3,5,6-tetramethylbenzenesulfonyl chloride (33 mg, 0.138 mmol). The reaction mixture was stirred at room temperature overnight. Further 2,3,5,6-tetramethyl-benzenesulfonyl chloride (33 mg, 0.138 mmol) and pyridine (12 μL, 0.15 mmol) were added. The reaction mixture was stirred for 2 days at room temperature and then $NaHCO_3$ (5 mL, saturated aqueous solution) was added. After stirring for 10 min at room temperature the reaction mixture was extracted with dichloromethane (20 mL). The organic phase was washed with HCl (aq., 2 M), dried over magnesium sulfate, filtered and then concentrated in vacuo. The title compound was obtained in

quantitative yield (80 mg) which was used in the next step without further purification.

59c) tert-butyl 3-[3-[(E)-oct-1-enyl]-4-[(2,3,5,6-tetramethylphenyl)sulfonylamino]-1-naphthyl]benzoate

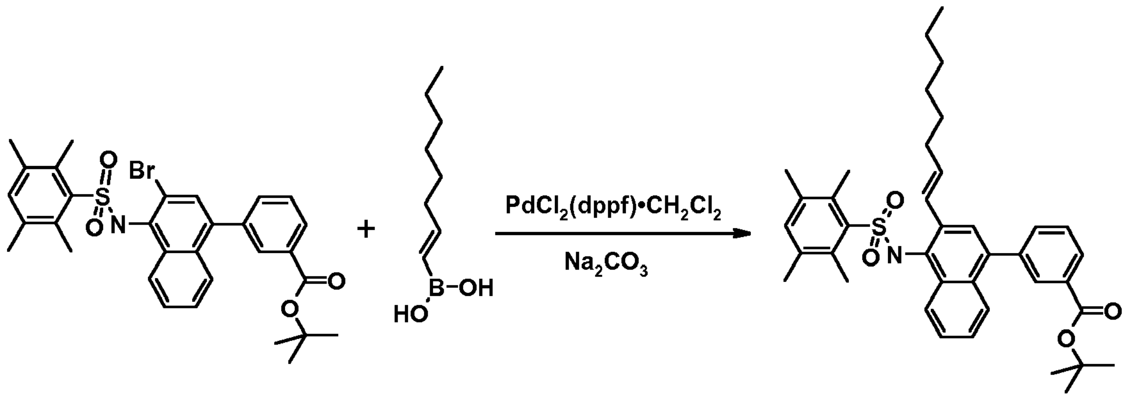


To a solution of tert-butyl 3-[3-bromo-4-[(2,3,5,6-tetramethylphenyl)-sulfonylamino]-1-naphthyl]benzoate (80 mg, 0.11 mmol) in dioxane (100 mL) were added (E)-oct-1-en-1-ylboronic acid (20 mg, 0.13 mmol), $PdCl_2(dppf)•CH_2Cl_2$ complex (5 mg, 0.05 mmol) and sodium carbonate (220 μL, 1mol/l aqueous solution). The reaction mixture was stirred at 90°C for 4 h. After cooling to room temperature, $H_2O$ (50 mL) was added, and the mixture was extracted with ethyl acetate. The combined organic phases were washed with $H_2O$, dried over sodium sulfate, filtered and then concentrated in vacuo. The resulting residue was purified via silica gel flash chromatography with n-heptane / ethyl acetate (3% to 20%) as eluent to yield 30 mg (45 %) of the title compound as a white powder.

59d) tert-butyl 3-[3-octyl-4-[(2,3,5,6-tetramethylphenyl)sulfonylamino]-1-naphthyl]-benzoate

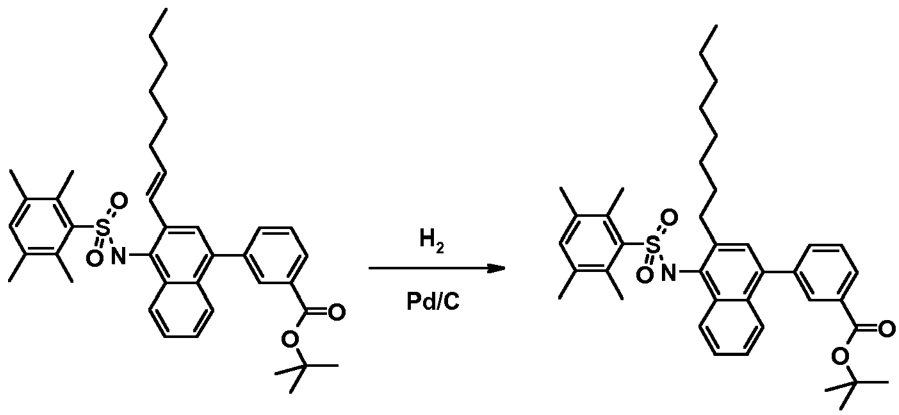


To a solution of tert-butyl 3-[3-[(E)-oct-1-enyl]-4-[(2,3,5,6-tetramethylphenyl)-sulfonylamino]-1-naphthyl]benzoate (30 mg, 0.48 mmol) in methanol (5 mL) were added Pd/C (5 mg, 0.048 mmol) and hydrogen (2 bar). The suspension was stirred at room temperature overnight. Filtration and removal of the solvent in vacuo afforded the title compound as colorless oil in 32 mg quantitative yield which was used in the next step without further purification.

59e) 3-[3-octyl-4-[(2,3,5,6-tetramethylphenyl)sulfonylamino]-1-naphthyl]benzoic acid

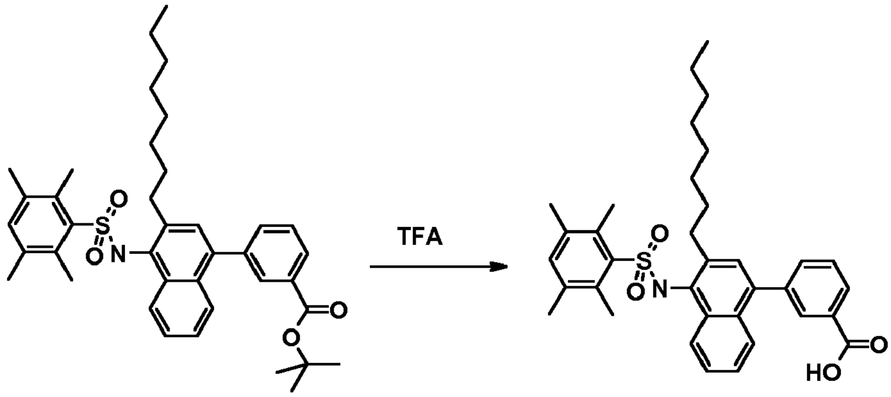


To a solution of tert-butyl 3-(2-octyl-4-(2,3,5,6-tetramethylphenylsulfonamido)-naphthalen-1-yl)benzoate (40 mg, 0.064 mmol) in dichloromethane (5 mL) was added trifluoroacetic acid (400 μl, 5.2 mmol). The reaction mixture was stirred at room temperature overnight and then concentrated in vacuo to yield 14 mg (37%) of the title compound.

Example 74:

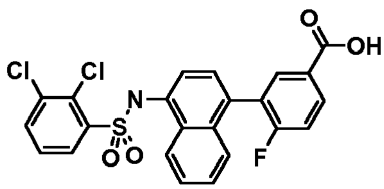

3-(4-(2,3-dichlorophenylsulfonamido)naphthalen-1-yl)-4-fluorobenzoic acid

74a) Methyl 3-(4-aminonaphthalen-1-yl)-4-fluorobenzoate

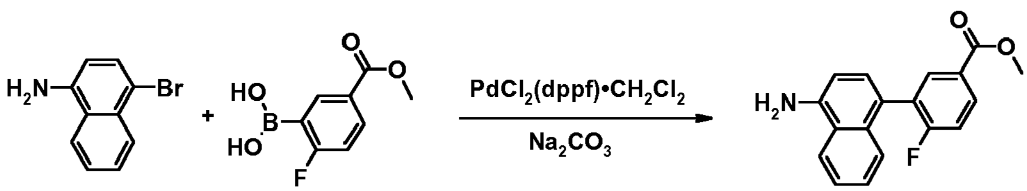


To a solution of 4-bromonaphthalen-1-amine (3.5 g, 15.76 mmol) in dioxane (100 mL) were added (2-fluoro-5-(methoxycarbonyl)-phenyl)boronic acid (3.12 g, 15.76 mmol), $PdCl_2$(dppf)•$CH_2Cl_2$ complex (650 mg, 0.79 mmol) and sodium carbonate (31.5 mL, 1mol/l aqueous solution). The reaction mixture was stirred at 90°C for 3 h. After cooling to room temperature, $H_2O$ (50 mL) was added, and the mixture was extracted with ethyl acetate. The combined organic phases were washed with $H_2O$, dried over sodium sulfate, filtered and then concentrated in vacuo. The resulting residue was purified via silica gel flash chromatography with n-heptane/ ethyl acetate (1:1) as eluent to yield 3.1 g (66 %) of the title compound as a white powder.

74b) Methyl 3-[4-[(2,3-dichlorophenyl)sulfonylamino]-1-naphthyl]-4-fluoro-benzoate

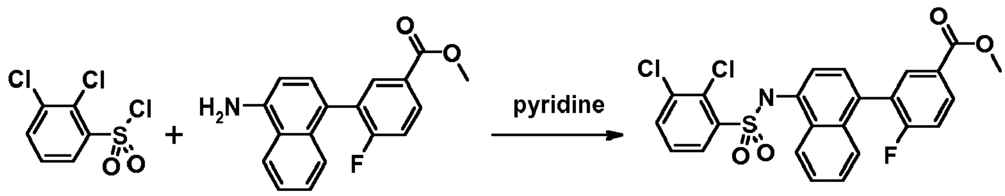


To a solution of methyl 3-(4-amino-1-naphthyl)-4-fluoro-benzoate (59 mg, 0.2 mmol) in dichloromethane (2 mL) were added pyridine (20 µL, 0.25 mmol) and 2,3-dichlorobenzenesulfonyl chloride (61 mg, 0.25 mmol). The reaction mixture was stirred at room temperature overnight. Afterwards the reaction mixture was diluted with dichloromethane (5 mL) and washed with hydrogen chloride (1 M aqueous solution), dried over magnesium sulfate, filtered and then concentrated in vacuo. Purification of the residue by high performance liquid chromatography (RP silica gel, acetonitrile/water/ trifluoroacetic acid) and lyophilization of the product fractions provided 41 mg (40 %) of the title compound as a white powder.

74c) 3-(4-(2,3-dichlorophenylsulfonamido)naphthalen-1-yl)-4-fluorobenzoic acid

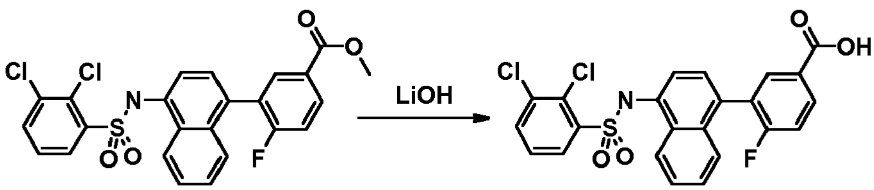


To a solution of methyl 3-[4-[(2,3-dichlorophenyl)sulfonylamino]-1-naphthyl]-4-fluorobenzoate (35 mg, 0.07 mmol) in a mixture of THF (2 mL), $H_2O$ (1.2 mL) and methanol (1 mL) was added lithium hydroxide (15 mg, 0.63 mmol). After stirring at room temperature overnight, the reaction mixture was diluted with THF (10 mL), neutralized with acetic acid, and concentrated in vacuo. Purification of the residue by high performance liquid chromatography (RP silica gel, acetonitrile/water/ trifluoroacetic acid) and lyophilization of the product fractions provided 25 mg (73 %) of the title compound as a white powder.

The compounds in the following table were prepared using standard chemical manipulations, readily available starting materials, and procedures similar to those used for the preparation of examples 1, 2, 5, 6, 8, 59, and 74.

Table 1:

| Ex. No. | Formula / IUPAC Name | IC50 [µM] | Prep | MS (M+1) | Rt (min) |
|---|---|---|---|---|---|
| 1 | 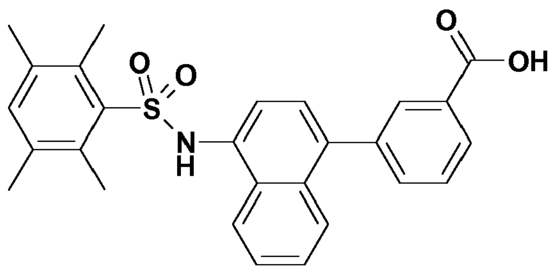 3-[4-[(2,3,5,6-tetramethyl-phenyl)-sulfonylamino]-1-naphthyl]benzoic acid | 0.2 | 1 | 460.09 | 1.9 |

(continued)

| Ex. No. | Formula / IUPAC Name | IC50 [μM] | Prep | MS (M+1) | Rt (min) |
|---|---|---|---|---|---|
| 2 | 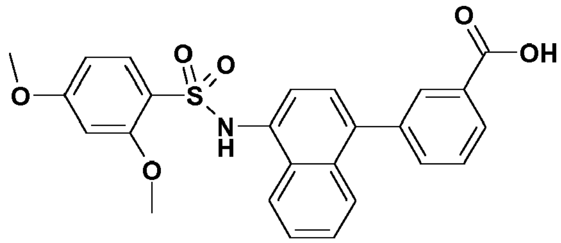 3-[4-[(2,4-dimethoxyphenyl)sulfonylamino]-1-naphthyl]benzoic acid | 4.5 | 2 | 464.3 | 1.73 |
| 3 | 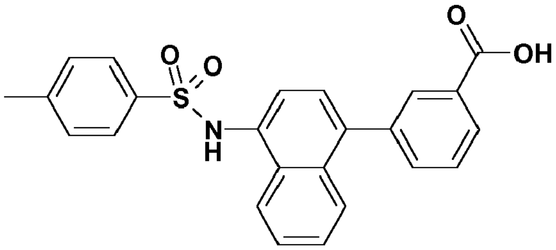 3-[4-(p-tolylsulfonylamino)-1-naphthyl]benzoic acid | 2.1 | 2 | 418.19 | 1.78 |
| 4 | 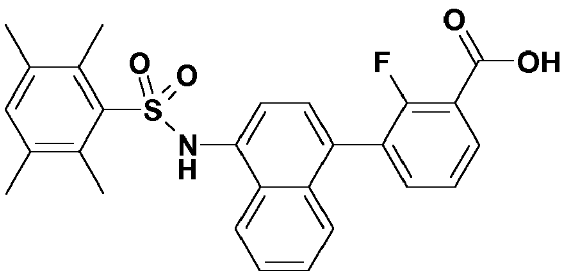 2-fluoro-3-[4-[(2,3,5,6-tetramethylphenyl)sulfonylamino]-1-naphthyl]benzoic acid | 0.2 | 5 | 478.27 | 1.9 |
| 5 | 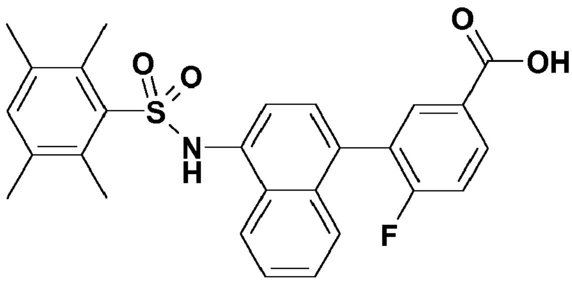 4-fluoro-3-[4-[(2,3,5,6-tetramethylphenyl)sulfonylamino]-1-naphthyl]benzoic acid | 0.09 | 5 | 478.27 | 1.91 |

(continued)

| Ex. No. | Formula / IUPAC Name | IC50 [μM] | Prep | MS (M+1) | Rt (min) |
|---|---|---|---|---|---|
| 6 | 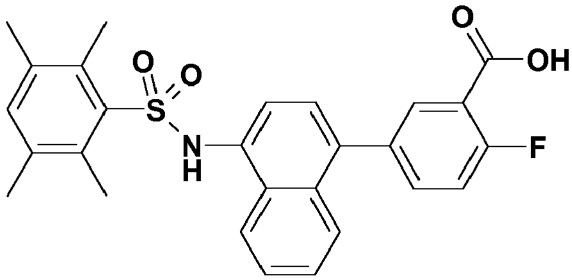 2-fluoro-5-[4-[(2,3,5,6-tetramethylphenyl)sulfonylamino]-1-naphthyl]benzoic acid | 1.7 | 6 | 478.35 | 1.91 |
| 7 | 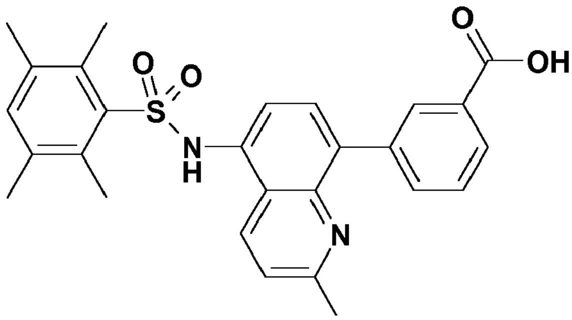 3-[2-methyl-5-[(2,3,5,6-tetramethylphenyl)sulfonylamino]-8-quinolyl]benzoic acid | 1.1 | 1 | 475.27 | 1.89 |
| 8 | 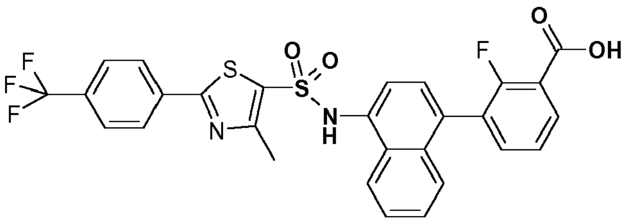 2-fluoro-3-[4-[[4-methyl-2-[4-(trifluoromethyl)phenyl]thiazol-5-yl]sulfonylamino]-1-naphthyl]benzoic acid | 5.2 | 8 | 587.3 | 2.04 |
| 9 | 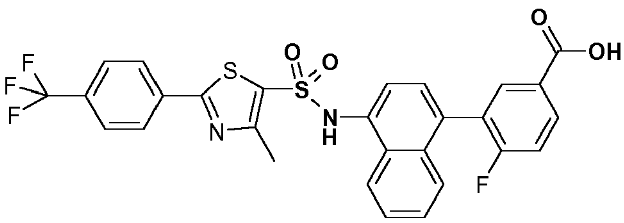 4-fluoro-3-[4-[[4-methyl-2-[4-(trifluoromethyl)phenyl]thiazol-5-yl]sulfonylamino]-1-naphthyl]benzoic acid | 5.8 | 8 | 587.28 | 2.06 |

(continued)

| Ex. No. | Formula / IUPAC Name | IC50 [μM] | Prep | MS (M+1) | Rt (min) |
|---|---|---|---|---|---|
| 10 | 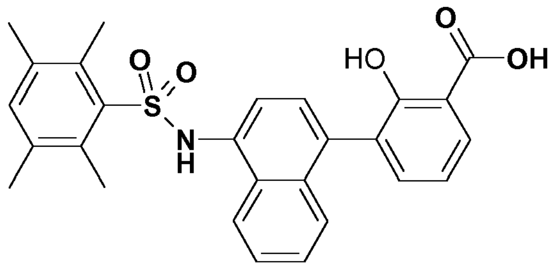 2-hydroxy-3-[4-[(2,3,5,6-tetramethylphenyl)sulfonylamino]-1-naphthyl]benzoic acid | 2.5 | 6 | 476.3 | 2 |
| 11 | 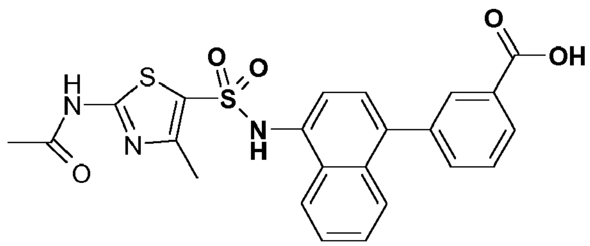 3-[4-[(2-acetamido-4-methyl-thiazol-5-yl)sulfonylamino]-1-naphthyl]benzoic acid | 2.8 | 2 | 482.28 | 1.7 |
| 12 | 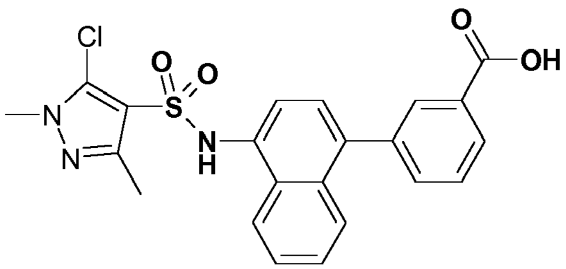 3-[4-[(5-chloro-1,3-dimethyl-pyrazol-4-yl)sulfonylamino]-1-naphthyl]benzoic acid | 0.4 | 2 | 456.26 | 1.76 |
| 13 | 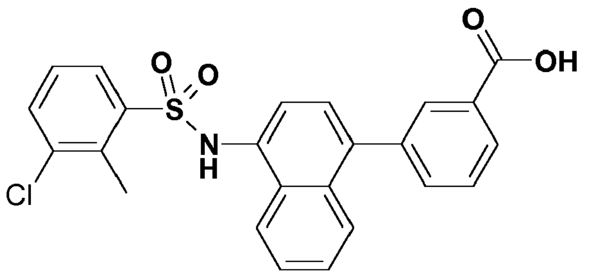 3-[4-[(3-chloro-2-methylphenyl)sulfonylamino]-1-naphthyl]benzoic acid | 1.1 | 2 | 450.31 | 1.96 |

(continued)

| Ex. No. | Formula / IUPAC Name | IC50 [µM] | Prep | MS (M+1) | Rt (min) |
|---|---|---|---|---|---|
| 14 | 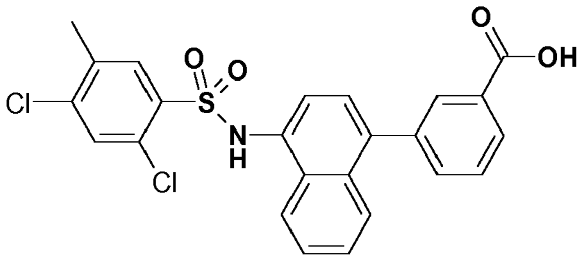 3-[4-[(2,4-dichloro-5-methylphenyl)sulfonylamino]-1-naphthyl]benzoic acid | 3.8 | 2 | 486.13 | 2.01 |
| 15 | 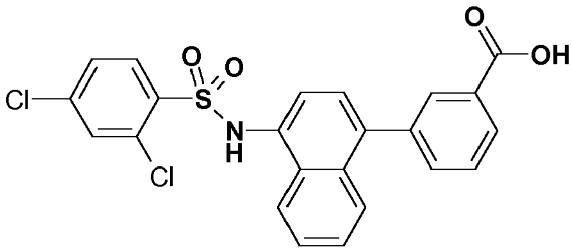 3-[4-[(2,4-dichlorophenyl)sulfonylamino]-1-naphthyl]benzoic acid | 1 | 2 | 470.3 | 1.96 |
| 16 | 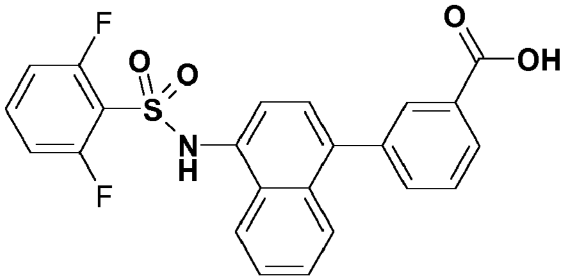 3-[4-[(2,6-difluorophenyl)sulfonylamino]-1-naphthyl]benzoic acid | 6.5 | 2 | 440.25 | 1.81 |
| 17 | 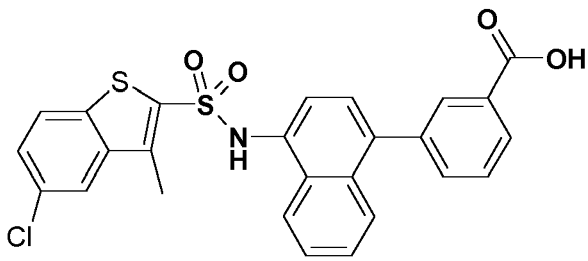 3-[4-[(5-chloro-3-methyl-benzothiophen-2-yl)sulfonylamino]-1-naphthyl]benzoic acid | 1.6 | 2 | 508.25 | 2.03 |
| 18 | 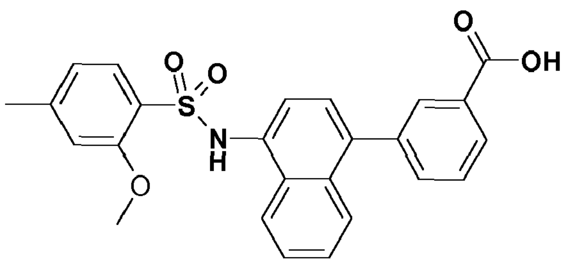 3-[4-[(2-methoxy-4-methylphenyl)sulfonylamino]-1-naphthyl]benzoic acid | 5.8 | 2 | 448.33 | 1.86 |

(continued)

| Ex. No. | Formula / IUPAC Name | IC50 [μM] | Prep | MS (M+1) | Rt (min) |
|---|---|---|---|---|---|
| 19 | 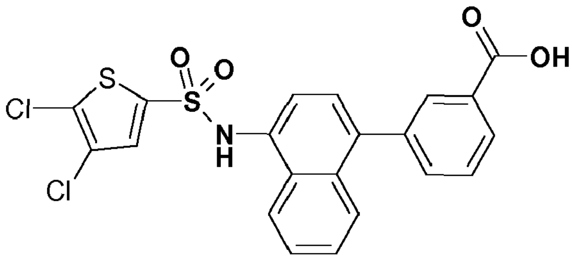 3-[4-[(4,5-dichloro-2-thienyl)sulfonylamino]-1-naphthyl]benzoic acid | 7 | 2 | 476.24 | 1.99 |
| 20 | 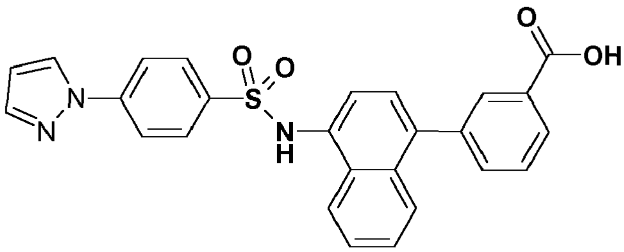 3-[4-[(4-pyrazol-1-ylphenyl)sulfonylamino]-1-naphthyl]benzoic acid | 4 | 2 | 470.33 | 1.82 |
| 21 | 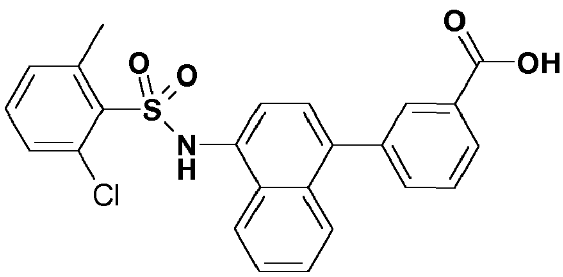 3-[4-[(2-chloro-6-methylphenyl)sulfonylamino]-1-naphthyl]benzoic acid | 1.8 | 2 | 450.29 | 1.92 |
| 22 | 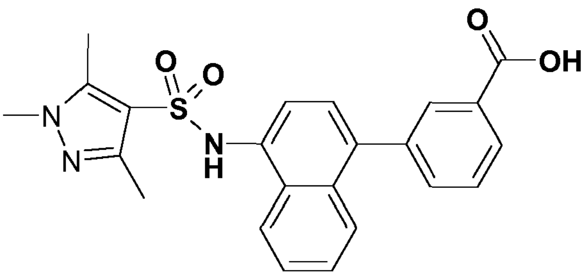 3-[4-[(1,3,5-trimethylpyrazol-4-yl)sulfonylamino]-1-naphthyl]benzoic acid | 0.8 | 2 | 436.3 | 1.7 |
| 23 | 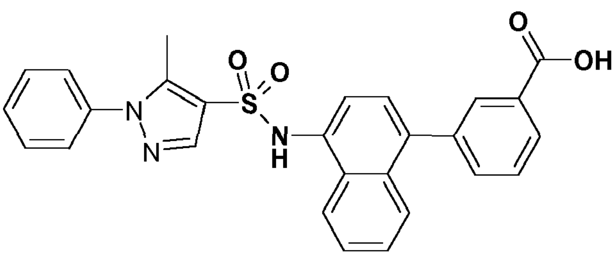 3-[4-[(5-methyl-1-phenyl-pyrazol-4-yl)sulfonylamino]-1-naphthyl]benzoic acid | 4.5 | 2 | 484.33 | 1.85 |

(continued)

| Ex. No. | Formula / IUPAC Name | IC50 [µM] | Prep | MS (M+1) | Rt (min) |
|---|---|---|---|---|---|
| 24 | 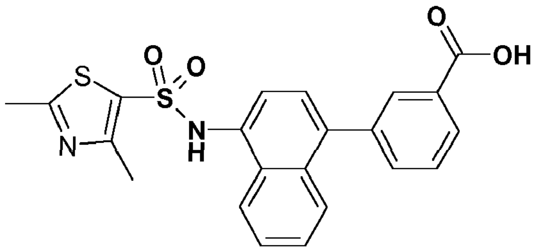 3-[4-[(2,4-dimethylthiazol-5-yl)sulfonylamino]-1-naphthyl]benzoic acid | 4.6 | 2 | 439.25 | 1.77 |
| 25 | 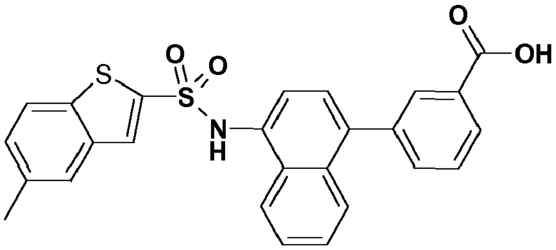 3-[4-[(5-methylbenzothiophen-2-yl)sulfonylamino]-1-naphthyl]benzoic acid | 3 | 2 | 474.31 | 1.98 |
| 26 | 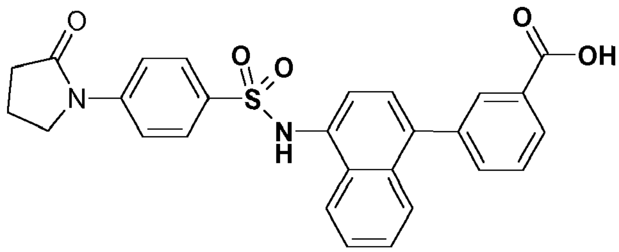 3-[4-[[4-(2-oxopyrrol id in-1-yl)phenyl]sulfonylamino]-1-naphthyl]benzoic acid | 2.5 | 2 | 487.33 | 1.75 |
| 27 | 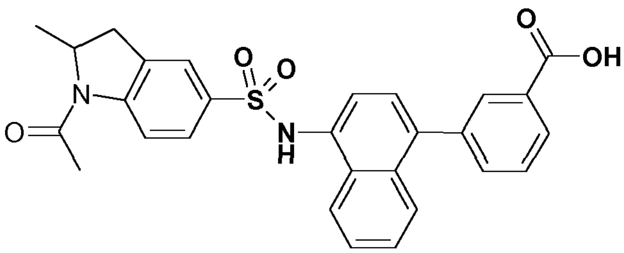 3-[4-[(1-acetyl-2-methyl-indolin-5-yl)sulfonylamino]-1-naphthyl]benzoic acid | 3.6 | 2 | 501.35 | 1.77 |

(continued)

| Ex. No. | Formula / IUPAC Name | IC50 [µM] | Prep | MS (M+1) | Rt (min) |
|---|---|---|---|---|---|
| 28 | 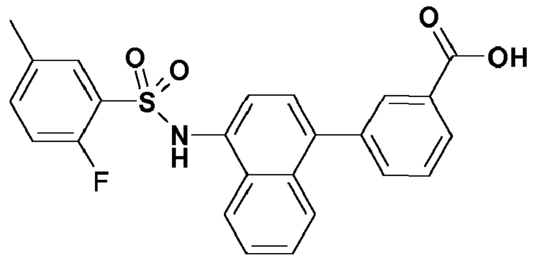 3-[4-[(2-fluoro-5-methylphenyl)sulfonylamino]-1-naphthyl]benzoic acid | 5.1 | 2 | 436.25 | 1.88 |
| 29 | 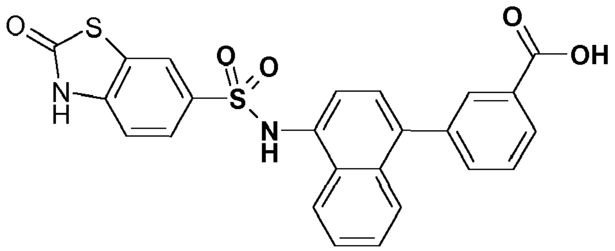 3-[4-[(2-oxo-3H-1,3-benzothiazol-6-yl)sulfonylamino]-1-naphthyl]benzoic acid | 5.1 | 2 | 477.21 | 1.72 |
| 30 | 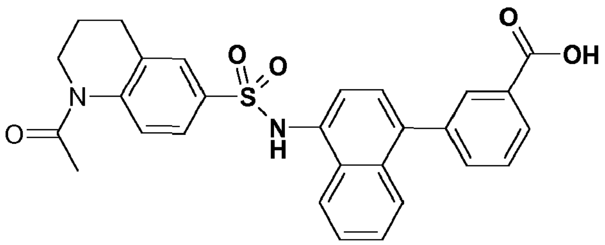 3-[4-[(1-acetyl-3,4-dihydro-2H-quinolin-6-yl)sulfonylamino]-1-naphthyl]benzoic acid | 4.3 | 2 | 501.34 | 1.76 |
| 31 | 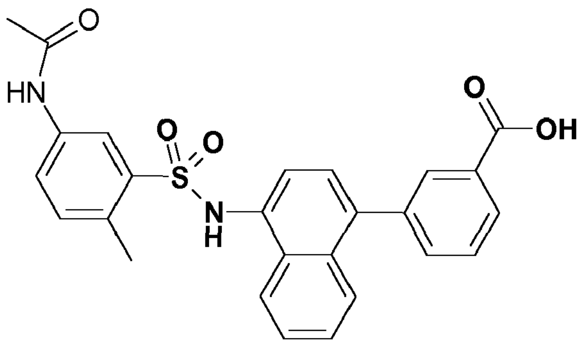 3-[4-[(5-acetamido-2-methylphenyl)sulfonylamino]-1-naphthyl]benzoic acid | 3.2 | 2 | 475.34 | 1.72 |

(continued)

| Ex. No. | Formula / IUPAC Name | IC50 [μM] | Prep | MS (M+1) | Rt (min) |
|---|---|---|---|---|---|
| 32 | 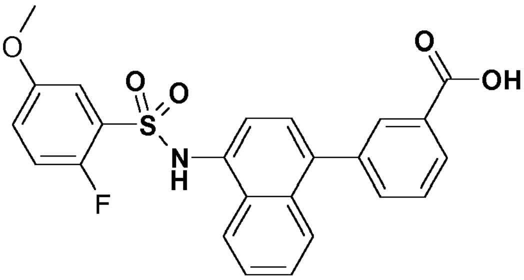 3-[4-[(2-fluoro-5-methoxyphenyl)sulfonylamino]-1-naphthyl]benzoic acid | 3.3 | 2 | 452.2 | 1.84 |
| 33 | 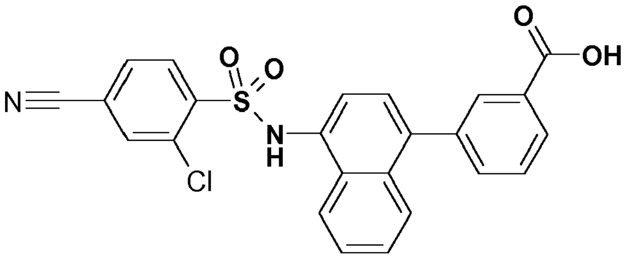 3-[4-[(2-chloro-4-cyanophenyl)sulfonylamino]-1-naphthyl]benzoic acid | 5.2 | 2 | 461.33 | 1.85 |
| 34 | 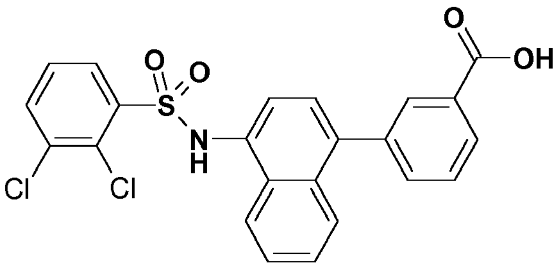 3-[4-[(2,3-dichlorophenyl)sulfonylamino]-1-naphthyl]benzoic acid | 1.5 | 2 | 470.35 | 1.94 |
| 35 | 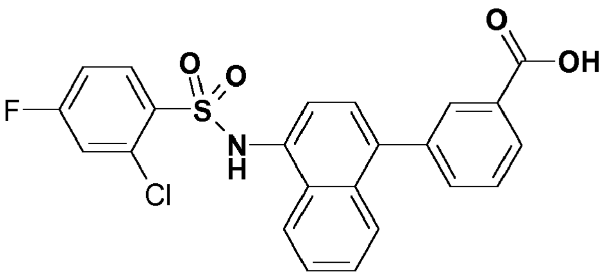 3-[4-[(2-chloro-4-fluorophenyl)sulfonylamino]-1-naphthyl]benzoic acid | 5.6 | 2 | 454.29 | 1.89 |

(continued)

| Ex. No. | Formula / IUPAC Name | IC50 [µM] | Prep | MS (M+1) | Rt (min) |
|---|---|---|---|---|---|
| 36 | 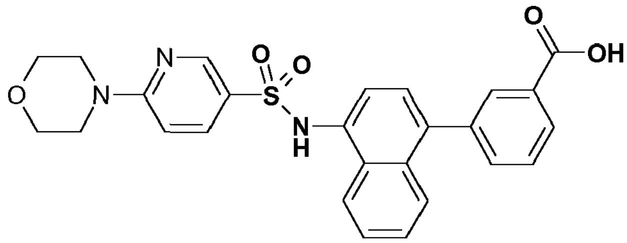<br>3-[4-[(6-morpholino-3-pyridyl)sulfonylamino]-1-naphthyl]benzoic acid | 4.2 | 2 | 490.36 | 1.76 |
| 37 | 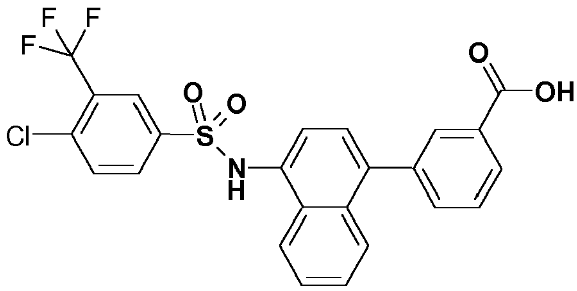<br>3-<br>[4-[[4-chloro-3-(trifluoromethyl)phenyl]sulfonylamino]-1-naphthyl]benzoic acid | 4 | 2 | 504.35 | 1.99 |
| 38 | 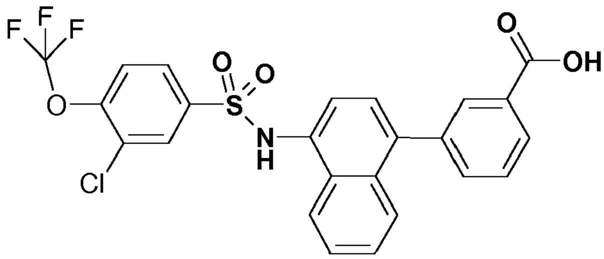<br>3-[4-[[3-chloro-4-(trifluoromethoxy)phenyl]sulfonylamino]-1-naphthyl]benzoic acid | 6.8 | 2 | 520.34 | 2.01 |
| 39 | 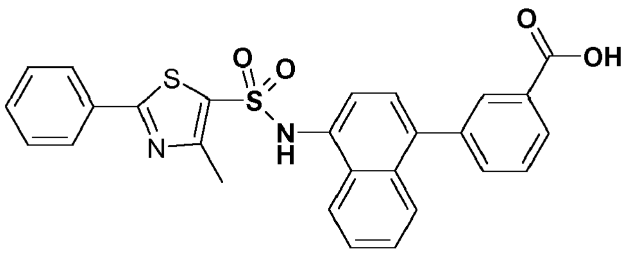<br>3-[4-[(4-methyl-2-phenyl-thiazol-5-yl)sulfonylamino]-1-naphthyl]benzoic acid | 2.8 | 2 | 501.3 | 1.98 |

(continued)

| Ex. No. | Formula / IUPAC Name | IC50 [μM] | Prep | MS (M+1) | Rt (min) |
|---|---|---|---|---|---|
| 40 | 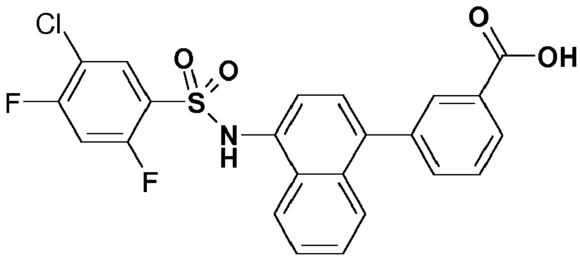 3-[4-[(5-chloro-2,4-difluorophenyl)sulfonylamino]-1-naphthyl]benzoic acid | 4.4 | 2 | 472.37 | 1.93 |
| 41 | 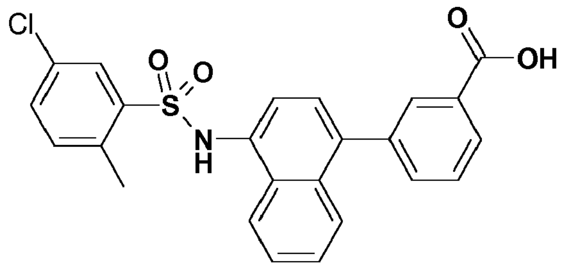 3-[4-[(5-chloro-2-methylphenyl)sulfonylamino]-1-naphthyl]benzoic acid | 1.4 | 2 | 450.34 | 1.95 |
| 42 | 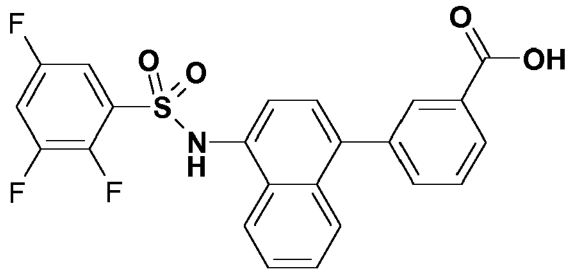 3-[4-[(2,3,5-trifluorophenyl)sulfonylamino]-1-naphthyl]benzoic acid | 6.4 | 2 | 456.31 | 1.89 |
| 43 | 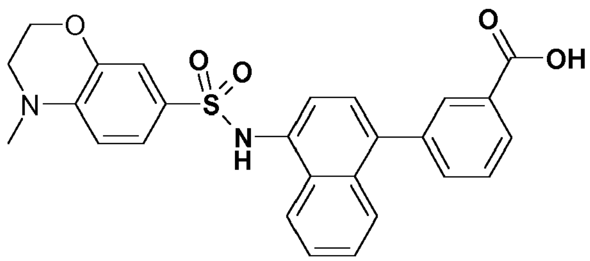 3-[4-[(4-methyl-2,3-d ihydro-1,4-benzoxazin-7-yl)sulfonylamino]-1-naphthyl]benzoic acid | 1.6 | 2 | 473.4 | 1.84 |
| 44 | 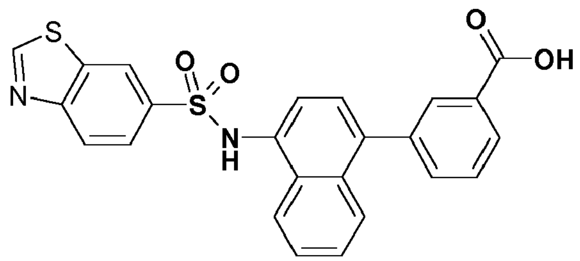 3-[4-(1,3-benzothiazol-6-ylsulfonylamino)-1-naphthyl]benzoic acid | 3.8 | 2 | 459.27 | 1.76 |

(continued)

| Ex. No. | Formula / IUPAC Name | IC50 [μM] | Prep | MS (M+1) | Rt (min) |
|---|---|---|---|---|---|
| 45 | 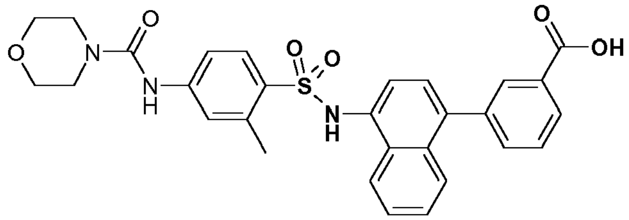 3-[4-[[2-methyl-4-(morpholine-4-carbonylamino)phenyl]sulfonylamino]-1-naphthyl]benzoic acid | 2.5 | 2 | 546.37 | 1.72 |
| 46 | 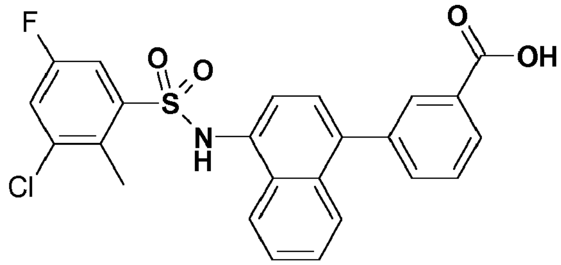 3-[4-[(3-chloro-5-fluoro-2-methylphenyl)sulfonylamino]-1-naphthyl]benzoic acid | 2.1 | 2 | 468.37 | 1.97 |
| 47 | 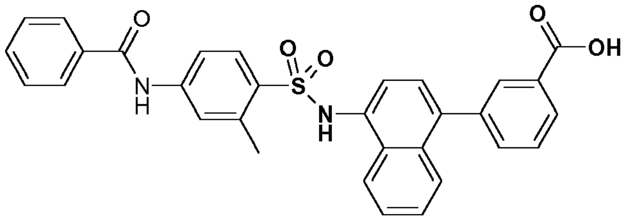 3-[4-[(4-benzamido-2-methylphenyl)sulfonylamino]-1-naphthyl]benzoic acid | 5 | 2 | 535.49 | 1.88 |
| 48 | 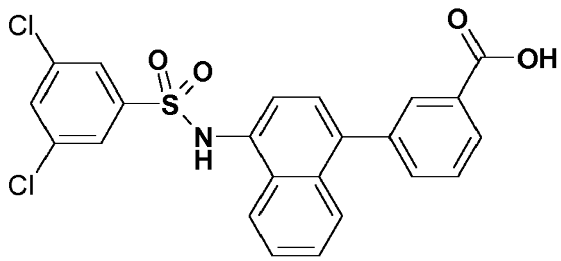 3-[4-[(3,5-dichlorophenyl)sulfonylamino]-1-naphthyl]benzoic acid | 2.3 | 2 | 470.17 | 1.91 |
| 49 | 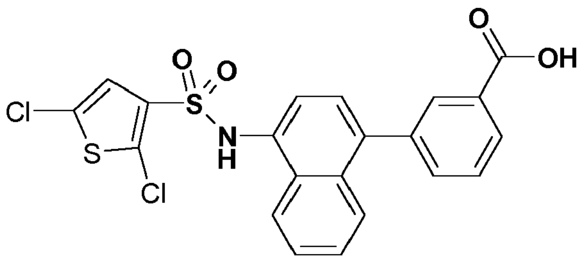 3-[4-[(2,5-dichloro-3-thienyl)sulfonylamino]-1-naphthyl]benzoic acid | 1.7 | 2 | 476.15 | 1.88 |

(continued)

| Ex. No. | Formula / IUPAC Name | IC50 [μM] | Prep | MS (M+1) | Rt (min) |
|---|---|---|---|---|---|
| 50 | 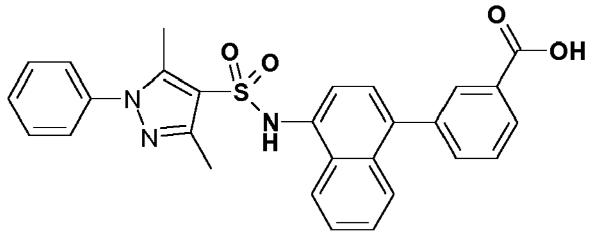<br>3-[4-[(3,5-dimethyl-1-phenyl-pyrazol-4-yl)sulfonylamino]-1-naphthyl]benzoic acid | 1.6 | 2 | 498.3 | 1.81 |
| 51 | 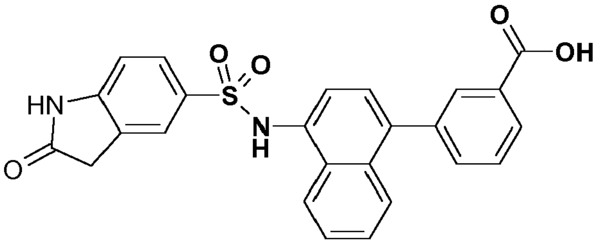<br>3-<br>[4-[(2-oxoindolin-5-yl)sulfonylamino]-1-naphthyl]benzoic acid | 7.8 | 2 | 459.25 | 1.58 |
| 52 | 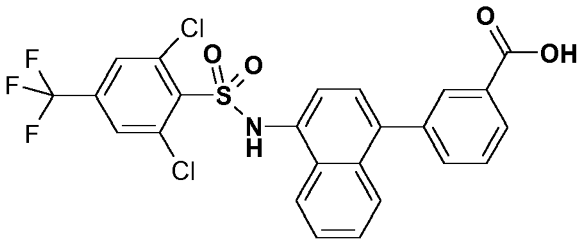<br>3-[4-[[2,6-dichloro-4-(trifluoromethyl)phenyl]sulfonylamino]-1-naphthyl]benzoic acid | 1.4 | 2 | 538.16 | 1.95 |
| 53 | 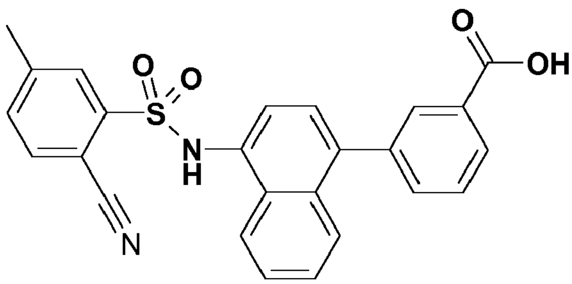<br>3-[4-[(2-cyano-5-methylphenyl)sulfonylamino]-1-naphthyl]benzoic acid | 6.7 | 2 | 441.18 | 1.76 |
| 54 | 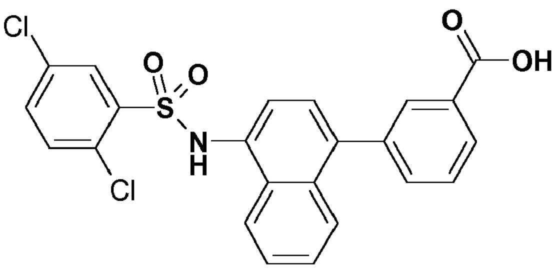<br>3-[4-[(2,5-dichlorophenyl)sulfonylamino]-1-naphthyl]benzoic acid | 3.7 | 2 | 470.16 | 1.88 |

(continued)

| Ex. No. | Formula / IUPAC Name | IC50 [μM] | Prep | MS (M+1) | Rt (min) |
|---|---|---|---|---|---|
| 55 | 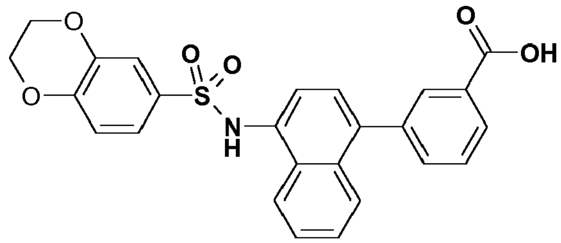 3-[4-(2,3-dihydro-1,4-benzodioxin-6-ylsulfonylamino)-1-naphthyl]benzoic acid | 4.3 | 2 | 462.26 | 1.74 |
| 56 | 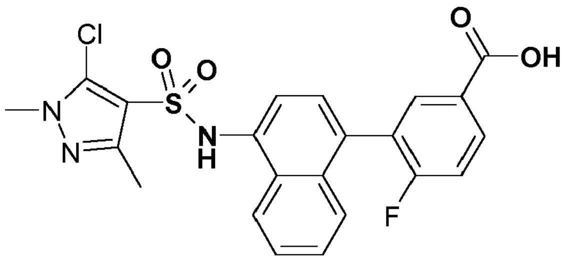 3-[4-[(5-chloro-1,3-dimethyl-pyrazol-4-yl)sulfonylamino]-1-naphthyl]-4-fluorobenzoic acid | 0.3 | 5 | 474.26 | 1.69 |
| 57 | 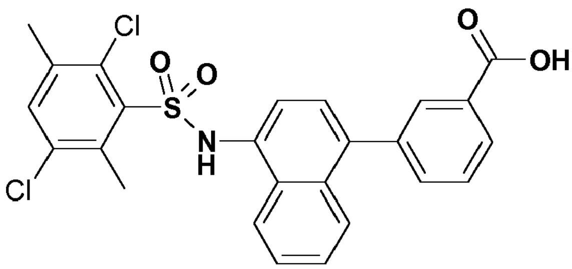 3-[4-[(2,5-dichloro-3,6-dimethylphenyl)sulfonylamino]-1-naphthyl]benzoic acid | 0.6 | 2 | 498.24 | 1.99 |
| 58 | 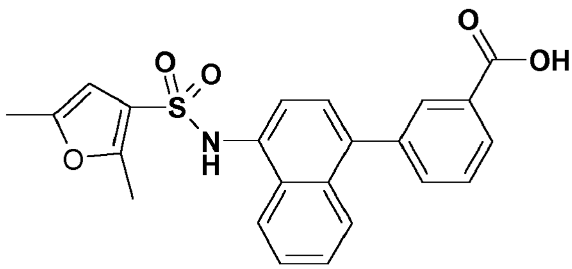 3-[4-[(2,5-dimethyl-3-furyl)sulfonylamino]-1-naphthyl]benzoic acid | 4.6 | 2 | 422.33 | 1.79 |

(continued)

| Ex. No. | Formula / IUPAC Name | IC50 [μM] | Prep | MS (M+1) | Rt (min) |
|---|---|---|---|---|---|
| 59 | 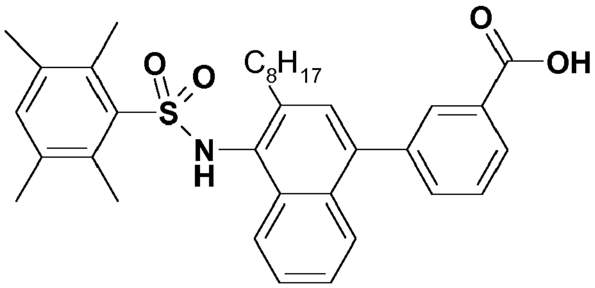 3-[3-octyl-4-[(2,3,5,6-tetramethylphenyl)sulfonylamino]-1-naphthyl]benzoic acid | 46 | 59 | 572.36 | 2.25 |
| 60 | 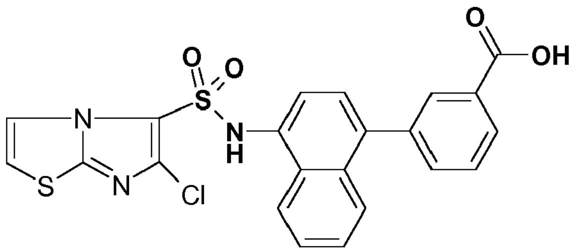 3-[4-[(6-chloroimidazo[2,1-b]thiazol-5-yl)sulfonylamino]-1-naphthyl]benzoic acid | 0.9 | 2 | 484.15 | 1.74 |
| 61 | 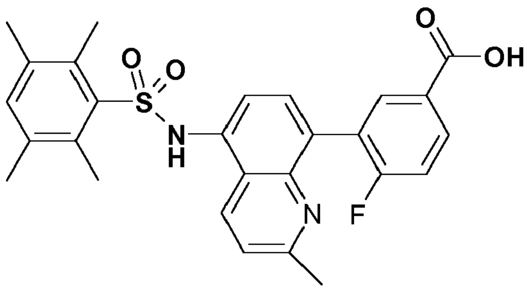 4-fluoro-3-[2-methyl-5-[(2,3,5,6-tetramethylphenyl)sulfonylamino]-8-quinolyl]benzoic acid; 2,2,2-trifluoroacetic acid | 0.5 | 74 | 493.26 | 1.9 |
| 62 | 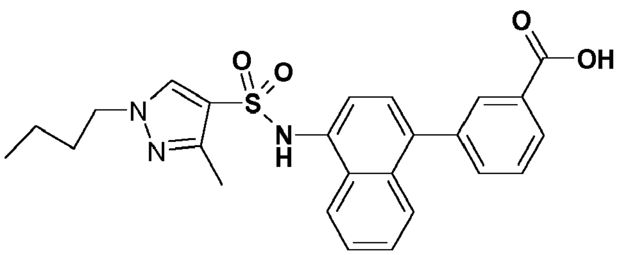 3-[4-[(1-butyl-3-methyl-pyrazol-4-yl)sulfonylamino]-1-naphthyl]benzoic acid | 0.7 | 2 | 464.36 | 1.78 |

(continued)

| Ex. No. | Formula / IUPAC Name | IC50 [μM] | Prep | MS (M+1) | Rt (min) |
|---|---|---|---|---|---|
| 63 | 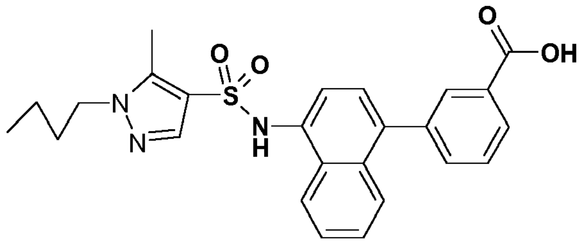<br>3-[4-[(5-methyl-1-propyl-pyrazol-4-yl)sulfonylamino]-1-naphthyl]benzoic acid | 3.2 | 2 | 450.29 | 1.71 |
| 64 | 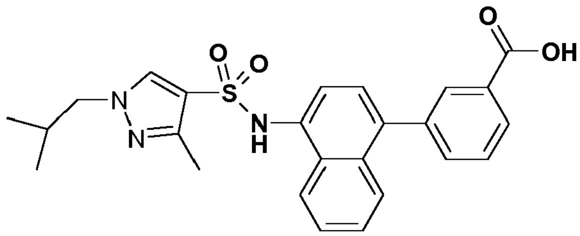<br>3-[4-[(1-isobutyl-3-methyl-pyrazol-4-yl)sulfonylamino]-1-naphthyl]benzoic acid | 4.5 | 2 | 464.26 | 1.73 |
| 65 | 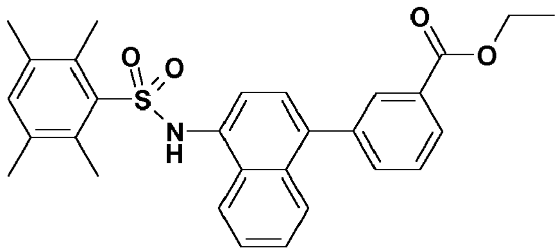<br>ethyl 3-[4-[(2,3,5,6-tetramethylphenyl)sulfonylamino]-1-naphthyl]benzoate | Prodrug | 6b | 488.2 | 2.16 |
| 66 | 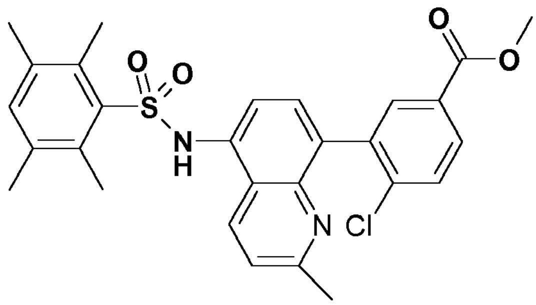<br>methyl 4-chloro-3-[2-methyl-5-[(2,3,5,6-tetramethylphenyl)sulfonylamino]-8-quinolyl]benzoate | Prodrug | 74b | 523.17 | 2.1 |

(continued)

| Ex. No. | Formula / IUPAC Name | IC50 [μM] | Prep | MS (M+1) | Rt (min) |
|---|---|---|---|---|---|
| 67 | 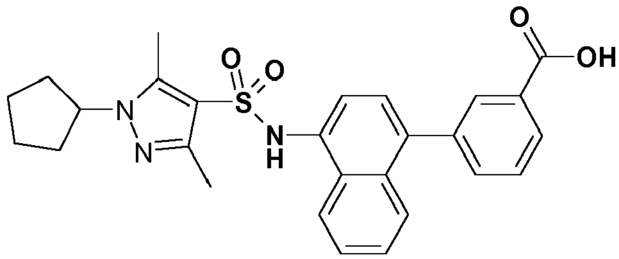 3-[4-[(1-cyclopentyl-5-methyl-pyrazol-4-yl)sulfonylamino]-1-naphthyl]benzoic acid | 1.8 | 74 | 476.13 | 1.8 |
| 68 | 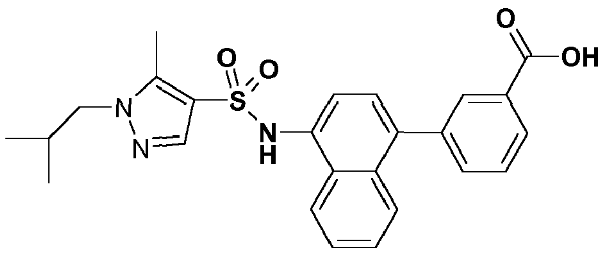 3-[4-[(1-isobutyl-5-methyl-pyrazol-4-yl)sulfonylamino]-1-naphthyl]benzoic acid | 2.4 | 74 | 464.17 | 1.78 |
| 69 | 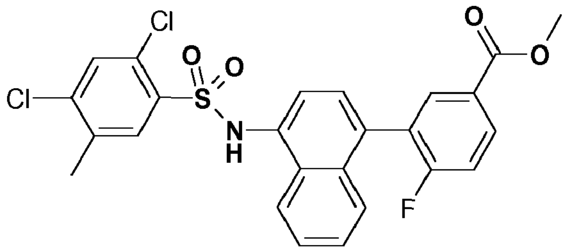 methyl 3-[4-[(2,4-dichloro-5-methylphenyl)sulfonylamino]-1-naphthyl]-4-fluorobenzoate | Prodrug | 74b | 518.11 | 2.08 |
| 70 | 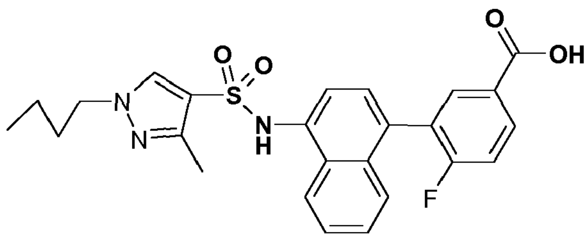 3-[4-[(1-butyl-3-methyl-pyrazol-4-yl)sulfonylamino]-1-naphthyl]-4-fluorobenzoic acid | 2.6 | 74 | 482.16 | 1.76 |

(continued)

| Ex. No. | Formula / IUPAC Name | IC50 [μM] | Prep | MS (M+1) | Rt (min) |
|---|---|---|---|---|---|
| 71 | 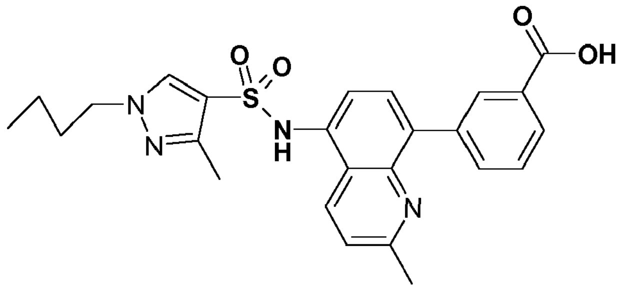 3-[5-[(1-butyl-3-methyl-pyrazol-4-yl)sulfonylamino]-2-methyl-8-quinolyl]benzoic acid | 22 | 74 | 479.15 | 1.7 |
| 72 | 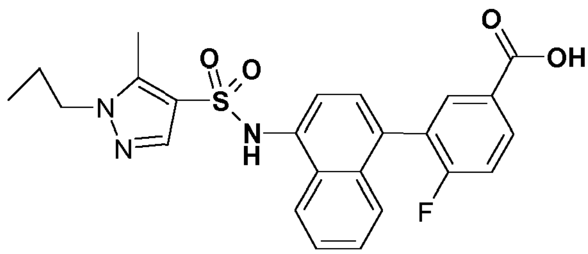 4-fluoro-3-[4-[(5-methyl-1-propyl-pyrazol-4-yl)sulfonylamino]-1-naphthyl]benzoic acid | 1.4 | 6 | 468.19 | 1.72 |
| 73 | 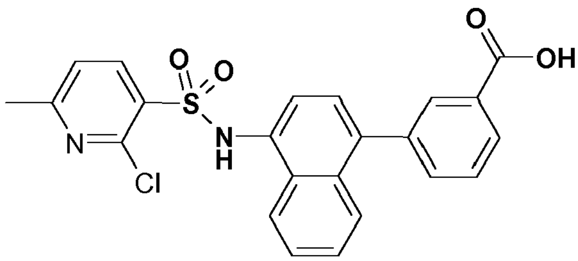 3-[4-[(2-chloro-6-methyl-3-pyridyl)sulfonylamino]-1-naphthyl]benzoic acid | 7.7 | 74 | 453.08 | 1.75 |
| 74 | 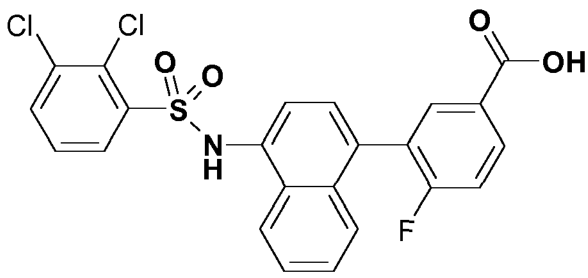 3-[4-[(2,3-dichlorophenyl)sulfonylamino]-1-naphthyl]-4-fluoro-benzoic acid | 0.9 | 74 | 488.08 | 1.89 |

(continued)

| Ex. No. | Formula / IUPAC Name | IC50 [µM] | Prep | MS (M+1) | Rt (min) |
|---|---|---|---|---|---|
| 75 | 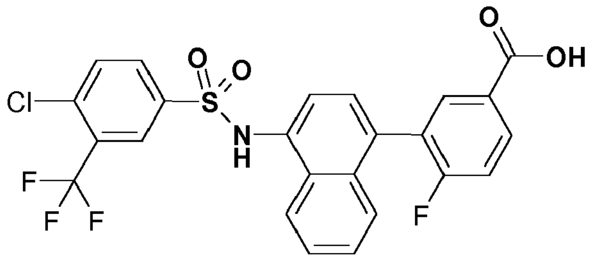 3-[4-[[4-chloro-3-(trifluoromethyl)phenyl]sulfonylamino]-1-naphthyl]-4-fluoro-benzoic acid | 4.4 | 74 | 524.01 | 1.94 |
| 76 | 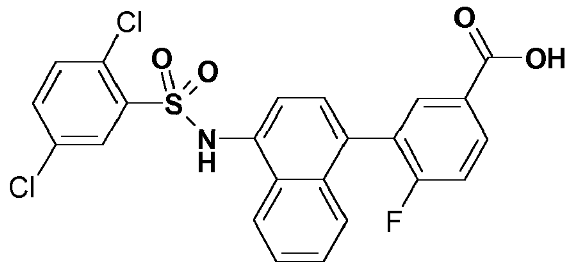 3-[4-[(2,5-dichlorophenyl)sulfonylamino]-1-naphthyl]-4-fluoro-benzoic acid | 2.4 | 74 | 489.94 | 1.9 |
| 77 | 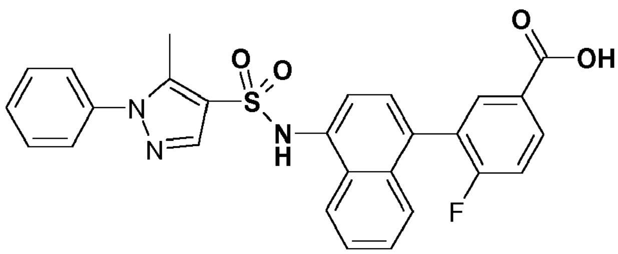 4-fluoro-3-[4-[(5-methyl-1-phenyl-pyrazol-4-yl)sulfonylamino]-1-naphthyl]benzoic acid | 3.7 | 74 | 502.11 | 1.82 |
| 78 | 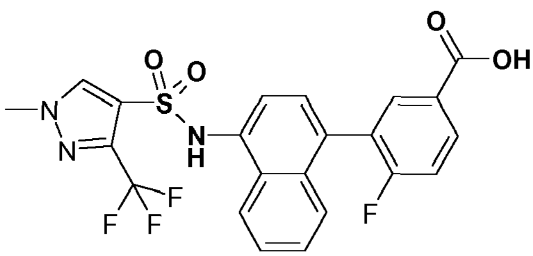 4-fluoro-3-[4-[[1-methyl-3-(trifluoromethyl)pyrazol-4-yl]sulfonylamino]-1-naphthyl]benzoic acid | 9.7 | 74 | 494.07 | 1.78 |

(continued)

| Ex. No. | Formula / IUPAC Name | IC50 [μM] | Prep | MS (M+1) | Rt (min) |
|---|---|---|---|---|---|
| 79 | 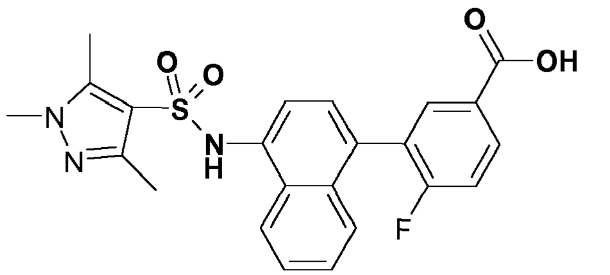 4-fluoro-3-[4-[(1,3,5-trimethylpyrazol-4-yl)sulfonylamino]-1-naphthyl]benzoic acid | 0.4 | 74 | 454.1 | 1.68 |
| 80 | 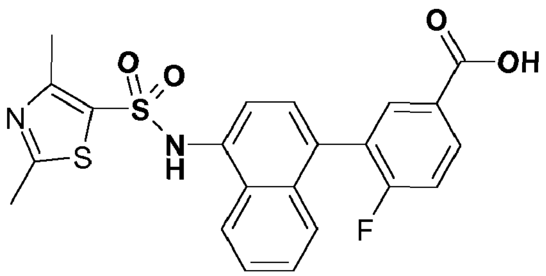 3-[4-[(2,4-dimethylthiazol-5-yl)sulfonylamino]-1-naphthyl]-4-fluorobenzoic acid | 1.9 | 74 | 457.04 | 1.74 |
| 81 | 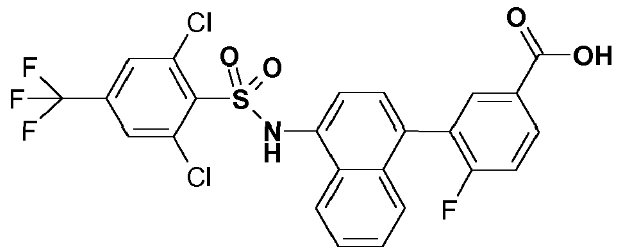 3-[4-[[2,6-dichloro-4-(trifluoromethyl)phenyl]sulfonylamino]-1-naphthyl]-4-fluoro-benzoic acid | 0.7 | 74 | 555.99 | 1.93 |
| 82 | 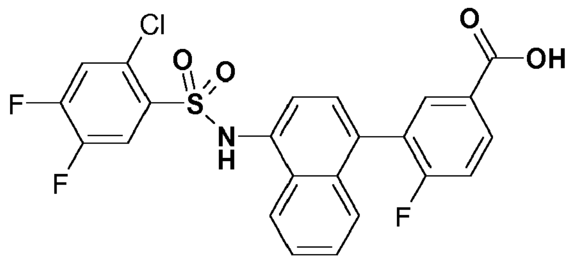 3-[4-[(2-chloro-4,5-difluorophenyl)sulfonylamino]-1-naphthyl]-4-fluorobenzoic acid | 5.1 | 74 | 492.07 | 1.88 |

(continued)

| Ex. No. | Formula / IUPAC Name | IC50 [μM] | Prep | MS (M+1) | Rt (min) |
|---|---|---|---|---|---|
| 83 | 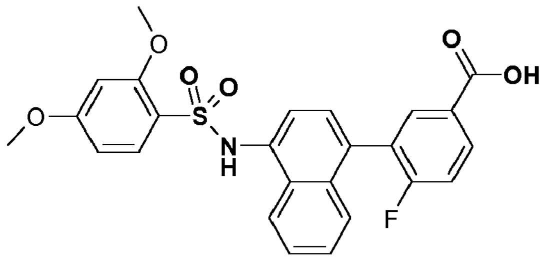 3-[4-[(2,4-dimethoxyphenyl)sulfonylamino]-1-naphthyl]-4-fluoro-benzoic acid | 2.2 | 74 | 482.09 | 1.8 |
| 84 | 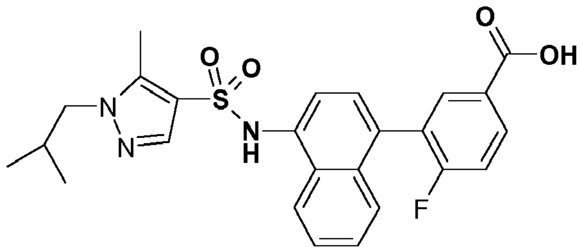 4-fluoro-3-[4-[(1-isobutyl-5-methyl-pyrazol-4-yl)sulfonylamino]-1-naphthyl]benzoic acid | 1.7 | 74 | 482.15 | 1.83 |
| 85 | 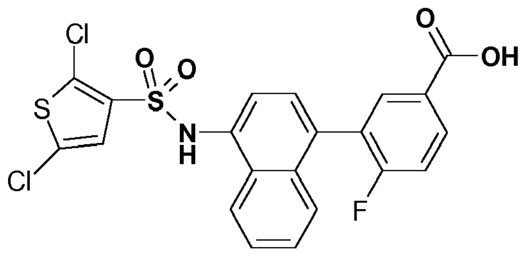 3-[4-[(2,5-dichloro-3-thienyl)sulfonylamino]-1-naphthyl]-4-fluoro-benzoic acid | 0.9 | 74 | 495.99 | 1.94 |
| 86 | 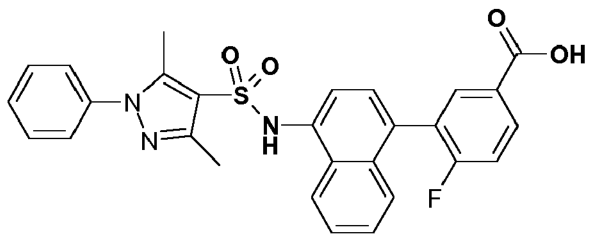 3-[4-[(3,5-dimethyl-1-phenyl-pyrazol-4-yl)sulfonylamino]-1-naphthyl]-4-fluorobenzoic acid | 1.5 | 74 | 516.16 | 1.86 |
| 87 | 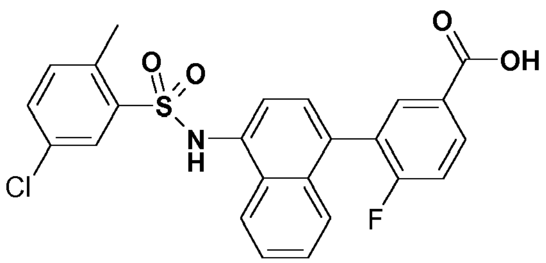 3-[4-[(5-chloro-2-methylphenyl)sulfonylamino]-1-naphthyl]-4-fluorobenzoic acid | 1 | 74 | 468.11 | 1.93 |

(continued)

| Ex. No. | Formula / IUPAC Name | IC50 [μM] | Prep | MS (M+1) | Rt (min) |
|---|---|---|---|---|---|
| 88 | 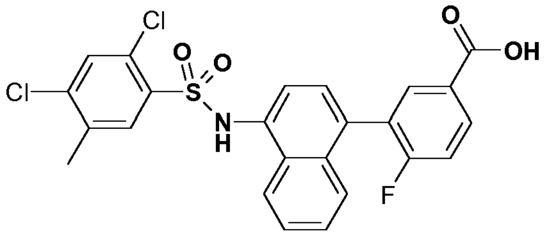 3-[4-[(2,4-dichloro-5-methylphenyl)sulfonylamino]-1-naphthyl]-4-fluorobenzoic acid | 1.3 | 74 | 504 | 1.99 |
| 89 | 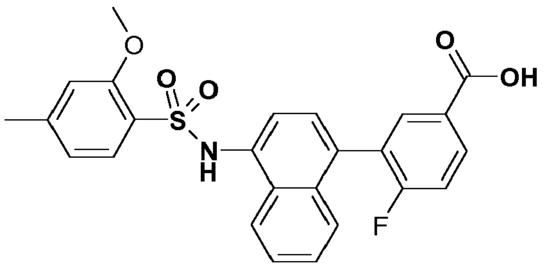 4-fluoro-3-[4-[(2-methoxy-4-methylphenyl)sulfonylamino]-1-naphthyl]benzoic acid | 2.5 | 74 | 466.14 | 1.85 |
| 90 | 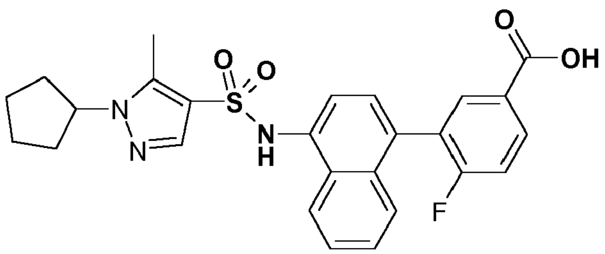 3-[4-[(1-cyclopentyl-5-methyl-pyrazol-4-yl)sulfonylamino]-1-naphthyl]-4-fluorobenzoic acid | 1.2 | 74 | 494.17 | 1.88 |
| 91 | 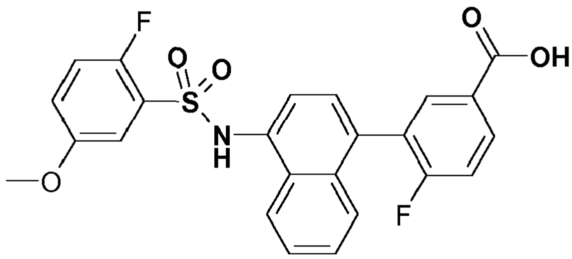 4-fluoro-3-[4-[(2-fluoro-5-methoxyphenyl)sulfonylamino]-1-naphthyl]benzoic acid | 2.2 | 74 | 470.15 | 1.74 |

(continued)

| Ex. No. | Formula / IUPAC Name | IC50 [µM] | Prep | MS (M+1) | Rt (min) |
|---|---|---|---|---|---|
| 92 | 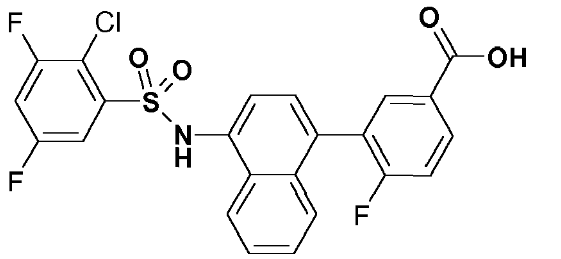 3-[4-[(2-chloro-3,5-difluorophenyl)sulfonylamino]-1-naphthyl]-4-fluorobenzoic acid | 5.3 | 74 | 490.09 | 1.9 |
| 93 | 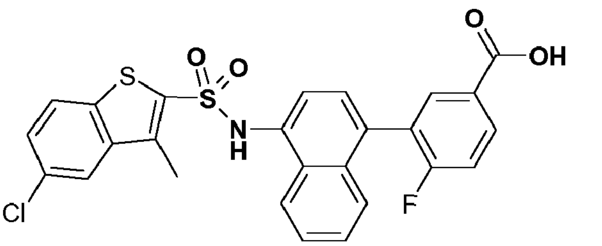 3-[5-[(5-chloro-3-methyl-benzothiophen-2-yl)sulfonylamino]-8-quinolyl]-4-fluoro-benzoic acid | 6.5 | 74 | 526.97 | 1.85 |
| 94 | 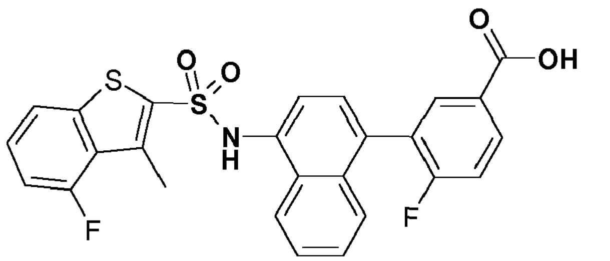 4-fluoro-3-[5-[(4-fluoro-3-methyl-benzothiophen-2-yl)sulfonylamino]-8-quinolyl]benzoic acid | 3 | 74 | 510.97 | 1.8 |
| 95 | 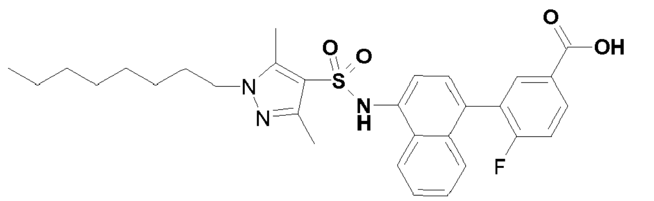 3-[5-[(3,5-dimethyl-1-octyl-pyrazol-4-yl)sulfonylamino]-8-quinolyl]-4-fluorobenzoic acid | 2.2 | 74 | 553.14 | 1.92 |
| 96 | 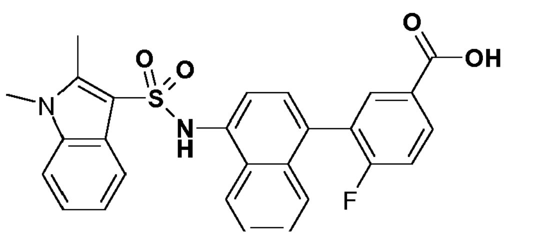 3-[5-[(1,2-dimethylindol-3-yl)sulfonylamino]-8-quinolyl]-4-fluoro-benzoic acid | 0.9 | 74 | 490.05 | 1.66 |

(continued)

| Ex. No. | Formula / IUPAC Name | IC50 [μM] | Prep | MS (M+1) | Rt (min) |
|---|---|---|---|---|---|
| 97 | 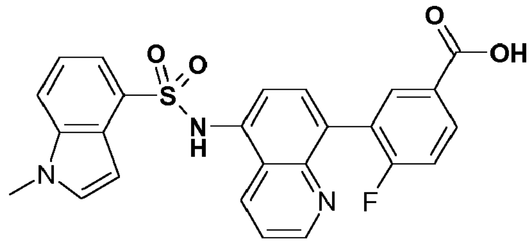 4-fluoro-3-[5-[(1-methylindol-4-yl)sulfonylamino]-8-quinolyl]benzoic acid | 5.5 | 74 | 476.05 | 1.62 |
| Prep: Preparation analog Ex. No. | | | | | |

Pharmacological Utility

The biological activity of the compounds of the invention may be demonstrated by known in vitro assays. Examples include in vitro cellular assays for recombinant and non-recombinant KEAP-1 as described above.
Based on the demonstrated ability of the compounds of the invention to inhibit KEAP-1 it is predicted that said compounds are useful for treatment of diseases and/or prevention of conditions which are modulated by KEAP-1.
Especially, the compounds of the invention may be useful to treat KEAP-1-related diseases and/or prevent KEAP-1-mediated conditions in humans.
The compounds of the invention are especially suitable for the treatment and/or prevention of:

1a) Disorders of fatty acid metabolism and glucose utilization disorders

1b) Disorders in which insulin resistance is involved

2) Diabetes mellitus, especially type 2 diabetes mellitus, including the prevention of the sequelae associated therewith. Particular aspects in this context are:

- a) Improvement of hyperglycemia
- b) Improvement of insulin resistance
- c) Improvement of glucose tolerance
- d) Protection of pancreatic beta cells
- e) Improvement of beta cell function
- f) Prevention of micro- and macrovascular disorders, such as
  - a. Retinopathy
  - b. Atherosclerosis
  - c. Nephropathy and microalbuminuria
  - d. Neuropathy
- g) Chronic low grade inflammation

3) Various other conditions which may be associated with the metabolic syndrome or the syndrome X, such as

- a) Increased abdominal girth
- b) Obesity
- c) Liver disorders
  - a. Fatty liver
  - b. Steatosis
  - c. Steatohepatitis

d. Cirrhosis

d) Dyslipidemia (e.g. hypertriglyceridemia, hypercholesterolemia, hyperlipoproteinemia and/or low HDL)
e) Insulin resistance
f) Hypercoagulability
g) Hyperuricemia
h) Thromboses, hypercoagulable and prothrombotic states (arterial and venous)
i) High blood pressure
j) Endothelial dysfunction
k) Heart failure, for example (but not limited to) following myocardial infarction, hypertensive heart disease or cardiomyopathy

4) Cardiovascular diseases, for example (but not limited to) myocardial infarction and stroke

5) Bone-related diseases and disorders characterized by redcued bone mass, such as:

a) Osteoporosis
b) Rheumatoid arthritis
c) Osteoarthritis.

6) Neurodegenerative diseases Such as

a) Multiple sclerosis
b) Parkinson's disease
c) Alzheimer's disease
d) Huntington's disease

7) Inflammatory diseases, CKD, COPD, asthma

8) Cancer

9) Fibrosis.

## Claims

**1.** A compound of the formula I

(I)

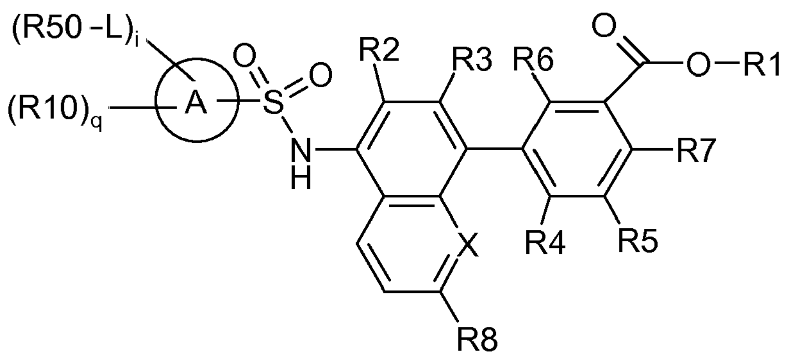

in which

R1 is H or ($C_1$-$C_6$)-alkyl;
A is phenyl, a 5-7 membered heteroaryl, a 9-11 membered heteroaryl or a 9 to 11 membered heterobicycle;
X is CH or N;

R2 is H, $(C_1-C_{16})$-alkyl or O-$(C_1-C_{16})$-alkyl;
R3 is H, $(C_1-C_{16})$-alkyl or O-$(C_1-C_{16})$-alkyl;
R4, R5, R6, R7 are independently of one another H, F, Cl, Br, I, OH, $CF_3$, $NO_2$, CN, $OCF_3$, O-$(C_1-C_6)$-alkyl, O-$(C_1-C_4)$-alkoxy-$(C_1-C_4)$-alkyl, S-$(C_1-C_6)$-alkyl, $(C_1-C_6)$-alkyl, N(R11)(R12), $SO_2$-$CH_3$, N(R13)CO(R14), N(R15)$SO_2$(R16) or CO(R17);
R8 is H or $(C_1-C_6)$-alkyl;
R10 is independently of each other F, Cl, Br, I, OH, $CF_3$, $NO_2$, CN, $OCF_3$, O-$(C_1-C_6)$-alkyl, O-$(C_1-C_4)$-alkoxy-$(C_1-C_4)$-alkyl, S-$(C_1-C_6)$-alkyl, $(C_1-C_8)$-alkyl, $(C_2-C_6)$-alkenyl, $(C_2-C_6)$-alkynyl, N(R18)(R19), $SO_2$-$CH_3$, COOH, COO-$(C_1-C_6)$-alkyl, CON(R20)(R21), N(R22)CO(R23), N(R24)$SO_2$(R25), CO(R26), $(C(R27)(R28))_z$-O(R29) or oxo;
z is 0, 1, 2, 3, 4, 5 or 6;
R11, R12, R13, R14, R15, R16, R17, R18, R19, R20, R21, R22, R23, R24, R25, R26,R27, R28, R29 are independently of one another H or $(C_1-C_6)$-alkyl;
q is 0, 1, 2, 3 or 4;
L is a bond, NH, O, CO, NHCO or CONH;
R50 is phenyl, a 5-6 membered heteroaryl, a 5-6 membered heterocyclyl or $(C_3-C_8)$-cycloalkyl, wherein R50 is optionally substituted one or more times independently of one another by F, Cl, Br, I, OH, $CF_3$, $NO_2$, CN, $OCF_3$, O-$(C_1-C_6)$-alkyl, O-$(C_1-C_4)$-alkoxy-$(C_1-C_4)$-alkyl, S-$(C_1-C_6)$-alkyl, $(C_1-C_6)$-alkyl, $(C_2-C_6)$-alkynyl, N(R31)(R32), $SO_2$-$CH_3$, COOH, COO-$(C_1-C_6)$-alkyl, CON(R33)(R34), N(R35)CO(R36), N(R37)$SO_2$(R38), CO(R39), $(C(R40)(R41))_y$-O(R42) or oxo;
R31, R32, R33, R34, R35, R36, R37, R38, R39, R40, R41, R42 are independently of one another H or $(C_1-C_6)$-alkyl;
y 0, 1, 2 ,3, 4, 5, 6;
i is 0 or 1;

in any of its stereoisomeric forms, or a mixture of stereoisomeric forms in any ratio, or a physiologically acceptable salt thereof, or a physiologically acceptable solvate of any of them.

**2.** A compound of the formula I as claimed in claim 1, wherein

X is CH.

**3.** A compound of the formula I as claimed in claim 1 or 2, wherein

X is N.

**4.** A compound of the formula I as claimed in any of claims 1 to 3, wherein

R2 is H;
R3 is H.

**5.** A compound of the formula I as claimed in any of claims 1 to 4, wherein

R2 is $(C_1-C_{16})$-alkyl or O-$(C_1-C_{16})$-alkyl;
R3 is H.

**6.** A compound of the formula I as claimed in any of claims 1 to 5, wherein

L is a bond.

**7.** A compound of the formula I as claimed in any of claims 1 to 6, wherein

R1 is H or $(C_1-C_6)$-alkyl;

A is selected from the group of phenyl, thiazol, pyrazol, thienyl, pyridyl, furyl, benzothiophen, 1,3-benzothiazol, imidazo[2,1b]thiazol, indol, indolin, di hydro-2H-quinolin, dihydro-1,4-benzoxazin, 2,3-dihydro-1,4-benzodioxin and tetraline;

X is CH or N;
R2 is H or ($C_1$-$C_8$)-alkyl;
R3 is H or ($C_1$-$C_8$)-alkyl;
R4, R5, R6 and R7 independently of one another H, F, Cl, OH, $CF_3$, CN, $OCF_3$, O-($C_1$-$C_6$)-alkyl or ($C_1$-$C_6$)-alkyl;
R8 is H or ($C_1$-$C_6$)-alkyl;
R10 is independently of each other F, Cl, Br, I, OH, $CF_3$, CN, $OCF_3$, O-($C_1$-$C_6$)-alkyl, ($C_1$-$C_8$)-alkyl, COOH, COO-($C_1$-$C_6$)-alkyl, N(R22)CO(R23), CO(R26), $(C(R27)(R28))_z$-O(R29) or oxo;
z is 0, 1, 2, 3 or 4;
R22, R23, R26, R27, R28, R29 are independently of one another H or ($C_1$-$C_6$)-alkyl.
q is 0, 1, 2, 3 or 4;
L is a bond, NHCO or CONH;
R50 is phenyl, pyrazol, pyrrolidin, morpholin or cyclopentyl, wherein R50 is optionally substituted once by $CF_3$ or oxo;
i is 0 or 1;

in any of its stereoisomeric forms, or a mixture of stereoisomeric forms in any ratio, or a physiologically acceptable salt thereof, or a physiologically acceptable solvate of any of them.

**8.** A compound of formula I as claimed in claim 1, selected from the series consiting of

4-fluoro-3-[4-[(2,3,5,6-tetramethylphenyl)sulfonylamino]-1-naphthyl]benzoic acid,
3-[4-[(2,3,5,6-tetramethylphenyl)sulfonylamino]-1-naphthyl]benzoic acid, 2-fluoro-3-[4-[(2,3,5,6-tetramethylphenyl)sulfonylamino]-1-naphthyl]benzoic acid,
3-[4-[(5-chloro-1,3-dimethyl-pyrazol-4-yl)sulfonylamino]-1-naphthyl]-4-fluoro-benzoic acid,
3-[4-[(5-chloro-1,3-dimethyl-pyrazol-4-yl)sulfonylamino]-1-naphthyl]benzoic acid,
4-fluoro-3-[4-[(1,3,5-trimethylpyrazol-4-yl)sulfonylamino]-1-naphthyl]benzoic acid,
4-fluoro-3-[2-methyl-5-[(2,3,5,6-tetramethylphenyl)sulfonylamino]-8-quinolyl]benzoic acid; 2,2,2-trifluoroacetic acid,
3-[4-[(2,5-dichloro-3,6-dimethyl-phenyl)sulfonylamino]-1-naphthyl]benzoic acid,
3-[4-[(1-butyl-3-methyl-pyrazol-4-yl)sulfonylamino]-1-naphthyl]benzoic acid,
3-[4-[[2,6-dichloro-4-(trifluoromethyl)phenyl]sulfonylamino]-1-naphthyl]-4-fluoro-benzoic acid,
3-[4-[(1,3,5-trimethylpyrazol-4-yl)sulfonylamino]-1-naphthyl]benzoic acid,
3-[4-[(2,3-dichlorophenyl)sulfonylamino]-1-naphthyl]-4-fluoro-benzoic acid,
3-[4-[(5-chloro-2-methyl-phenyl)sulfonylamino]-1-naphthyl]-4-fluoro-benzoic acid,
3-[4-[(2,4-dichloro-5-methyl-phenyl)sulfonylamino]-1-naphthyl]-4-fluoro-benzoic acid, and
3-[5-[(1,2-dimethylindol-3-yl)sulfonylamino]-8-quinolyl]-4-fluoro-benzoic acid;

in any of its stereoisomeric forms, or a mixture of stereoisomeric forms in any ratio, or a physiologically acceptable salt thereof, or a physiologically acceptable solvate of any of them.

**9.** A pharmaceutical composition comprising at least one compound as claimed in any of claims 1 to 8 or a physiologically acceptable salt of any of them, for use as a pharmaceutical.

**10.** A pharmaceutical composition as claimed in claim 9, comprising additionally one or more active ingredients selected from the series of:

Insulin and insulin derivatives, GLP-1, GLP-1 analogues and GLP-1 receptor agonists, polymer bound GLP-1 and GLP-1 analogues, dual GLP-1/GIP agonists, dual GLP-1/glucagon receptor agonists, PYY3-36 or analogues thereof, pancreatic polypeptide or analogues thereof, glucagon receptor agonists or antagonists, GIP receptor agonists or antagonists, ghrelin antagonists or inverse agonists, xenin and analogues thereof, DDP-IV inhibitors, SGLT-2 inhibitors, dual SGLT-2/SGLT-1 inhibitors, biguanides, thiazolidinediones, PPAR agonists, PPAR modulators, sulfonylureas, meglitinides, alpha-glucosidase inhibitors, amylin and amylin analogues, GPR119 agonists, GPR40 agonists, GPR120 agonists, GPR142 agonists, TGR5 agonists, AMPK stimulants, AMPK activators, inhibitors of 11-beta-HSD, activators of glucokinase, inhibitors of DGAT, inhibitors of protein tyrosine phosphatase 1, inhibitors of glucose-6-phosphatase, inhibitors of fructose-1,6-bisphosphatase, inhibitors of glycogen phosphorylase, inhibitors of phosphoenol pyruvate carboxykinase, inhibitors of glycogen synthase kinase, inhibitors of pyruvate dehydrogenase kinase, alpha2 adrenergic receptor antagonists, CCR-2 antagonists, modulators of glucose transporter-4, somatostatin receptor 3 agonists, HMG-CoA-reductase inhibitors,

fibrates, nicotinic acid and derivatives thereof, nicotinic acid receptor 1 agonists, , ACAT inhibitors, cholesterol absorption inhibitors, bile acid-binding substances, IBAT inhibitors, MTP inhibitors, modulators of PCSK9, LDL receptor up-regulators (liver selective thyroid hormone receptor beta agonists), HDL-raising compounds, lipid metabolism modulators, PLA2 inhibitors, ApoA-I enhancers, cholesterol synthesis inhibitors, omega-3 fatty acids and derivatives thereof, active substances for the treatment of obesity, CB1 receptor antagonists, MCH-1 antagonists, MC4 receptor agonists and partial agonists, NPY5 or NPY2 antagonists, NPY4 agonists, beta-3 adrenergic receptor agonists, leptin or leptin mimetics, 5HT2c receptor agonists, lipase inhibitors, angiogenesis inhibitors, H3 antagonists, AgRP inhibitors, triple monoamine uptake inhibitors, MetAP2 inhibitors, antisense oligonucleotides against production of fibroblast growth factor receptor 4 or prohibitin targeting peptide-1, drugs for influencing high blood pressure, chronic heart failure or atherosclerosis, angiotensin II receptor antagonists, ACE inhibitors, ECE inhibitors, diuretics, beta-blockers, calcium antagonists, centrally acting antihypertensives, antagonists of the alpha-2 adrenergic receptor, inhibitors of neutral endopeptidase and thrombocyte aggregation inhibitors, drugs for influencing high blood pressure, chronic heart failure or atherosclerosis, nitric oxide donors, angiotensin II receptor antagonists, dual ARB agonists, ACE inhibitors, ACE-2 activators, AT1 antagonists, AT2 receptor agonists, renin inhibitors, prorenin inhibitors, ECE inhibitors, endothelin receptor blockers, endothelin antagonists, diuretics, aldosterone antagonists, aldosterone synthase inhibitors, alpha-blockers, antagonists of the alpha-2 adrenergic receptor, beta-blockers, mixed alpha-/beta-blockers, calcium antagonists/calcium channel blockers, nasal formulations of calcium channel blockers, dual mineralocorticoid/CCBs, centrally acting antihypertensives, antagonists of the alpha-2 adrenergic receptor, inhibitors of neutral endopeptidase, aminopeptidase-A inhibitors, vasopeptide inhibitors, dual vasopeptide inhibitors, neprilysin-ACE inhibitors, neprilysin-ECE inhibitors, dual-acting AT receptor-neprilysin inhibitors, dual AT1/ETA antagonists, advanced glycation end-product breakers, recombinant renalase, blood pressure vaccines, anti-RAAS vaccines, AT1- or AT2-vaccines, modulators of genetic polymorphisms with antihypertensive response and thrombocyte aggregation inhibitors.

**11.** A compound of the formula I as claimed in any of claims 1 to 8 for use as a pharmaceutical.

**12.** A compound of the formula I as claimed in any of claims 1 to 8 for the prevention and/or treatment of diabetes, obesity, dyslipidemia and related disorders.

**13.** A compound of the formula I as claimed in any of claims 1 to 8 for the prevention and/or treatment of diabetes.

**14.** A compound of the formula I as claimed in any of claims 1 to 8 for the prevention and/or treatment of multiple sclerosis, Parkinson’s disease, Alzheimer’s disease and Huntington’s disease.

**15.** A compound of the formula I as claimed in any of claims 1 to 8 for the prevention and/or treatment of multiple sclerosis.

**16.** A compound of the formula I as claimed in any of claims 1 to 8 for prevention and/or treatment of a disease associated with the KEAP-1 modulation.

Europäisches Patentamt
European Patent Office
Office européen des brevets

# EUROPEAN SEARCH REPORT

Application Number
EP 14 30 6429

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2003/158202 A1 (CALDIROLA PATRIZIA [SE] ET AL) 21 August 2003 (2003-08-21)<br>* claims 1, 17, 18 *<br>* pages 7-15; examples 1-66; table 1 *<br>----- | 1-16 | INV.<br>C07D213/71<br>C07D215/36<br>C07D215/38<br>C07D231/12<br>C07D231/18<br>C07D333/34<br>C07D333/62<br>C07C209/30<br>C07C311/21<br>C07C311/29<br>C07C311/46<br>C07D265/36<br>C07D207/27<br>C07D277/36<br>C07D209/08<br>C07D277/46<br>C07D277/62 |
| A | US 2012/142917 A1 (LAWRENCE HARSHANI [US] ET AL) 7 June 2012 (2012-06-07)<br>* claims 1, 2 *<br>* page 29; table 1; compounds YG1-155 *<br>* page 1, paragraph 0004 *<br>----- | 1-16 | |
| A,D | CHUNLIN ZHUANG ET AL: "Rapid Identification of Keap1-Nrf2 Small-Molecule Inhibitors through Structure-Based Virtual Screening and Hit-Based Substructure Search", JOURNAL OF MEDICINAL CHEMISTRY, vol. 57, no. 3, 13 February 2014 (2014-02-13), pages 1121-1126, XP055170273, ISSN: 0022-2623, DOI: 10.1021/jm4017174<br>* abstract *<br>* page 1122; figures 1, 2 *<br>----- | 1-16 | TECHNICAL FIELDS SEARCHED (IPC)<br>C07D<br>C07C |

The present search report has been drawn up for all claims

1

| Place of search | Date of completion of the search | Examiner |
|---|---|---|
| The Hague | 20 February 2015 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 30 6429

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | | | C07D277/68<br>C07D209/34<br>C07D513/04<br>C07D295/215<br>C07D307/64 |
| | | | TECHNICAL FIELDS SEARCHED (IPC) |
| | | | |

The present search report has been drawn up for all claims

1

| Place of search | Date of completion of the search | Examiner |
|---|---|---|
| The Hague | 20 February 2015 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 14 30 6429

This annex lists the patent family members relating to the patent documents cited in the above-mentioned European search report. The members are as contained in the European Patent Office EDP file on
The European Patent Office is in no way liable for these particulars which are merely given for the purpose of information.

20-02-2015

| Patent document cited in search report | Publication date | Patent family member(s) | Publication date |
|---|---|---|---|
| US 2003158202 A1 | 21-08-2003 | AU 2002309435 B2 | 14-08-2008 |
| | | BR 0210291 A | 13-07-2004 |
| | | CA 2445653 A1 | 19-12-2002 |
| | | CN 1522245 A | 18-08-2004 |
| | | CN 1800185 A | 12-07-2006 |
| | | EP 1412325 A1 | 28-04-2004 |
| | | HK 1066790 A1 | 06-11-2009 |
| | | JP 4731114 B2 | 20-07-2011 |
| | | JP 2004536080 A | 02-12-2004 |
| | | JP 2010013473 A | 21-01-2010 |
| | | KR 20040007688 A | 24-01-2004 |
| | | KR 20080080172 A | 02-09-2008 |
| | | KR 20090019894 A | 25-02-2009 |
| | | MX PA03011083 A | 08-07-2004 |
| | | NZ 529032 A | 27-04-2007 |
| | | PL 367264 A1 | 21-02-2005 |
| | | US 2003158202 A1 | 21-08-2003 |
| | | US 2007066598 A1 | 22-03-2007 |
| | | US 2007066599 A1 | 22-03-2007 |
| | | US 2007066600 A1 | 22-03-2007 |
| | | US 2010222330 A1 | 02-09-2010 |
| | | WO 02100822 A1 | 19-12-2002 |
| | | YU 98003 A | 03-03-2006 |
| US 2012142917 A1 | 07-06-2012 | US 2012142917 A1 | 07-06-2012 |
| | | WO 2010102286 A2 | 10-09-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013067036 A
- CN 101836971 A


### Non-patent literature cited in the description


- **D. MARCOTTE et al.** *Bioorganic and Medical Chemistry,* 2013, vol. 21, 4011-4019
- **ZHUANG et al.** *J. Med. Chem.,* 2014, vol. 57, 1121-1126
- USP Dictionary of USAN and International Drug Names, US Pharmacopeia, Rockville. 2012
- *Pharmaceutical Research,* 1986, vol. 2 (6), 318